# NEW EUROPEAN PATENT SPECIFICATION

(11) **EP 0 551 401 B2**
(45) Date of publication and mention of the opposition decision: **01.12.2004**
(45) Mention of the grant of the patent: 02.11.1995
(21) Application number: 91918778.1
(22) Date of filing: 12.09.1991
(51) Int. Cl.: C12N 15/85, A61K 48/00, A61K 35/12, C12N 5/10, C12N 15/19, C12N 15/31, C12N 15/54

(54) **METHODS AND COMPOSITIONS FOR GENETIC THERAPY AND POTENTIATION OF ANTI-TUMOR IMMUNITY**
VERFAHREN UND ZUSAMMENSETZUNGEN FÜR GENTHERAPIE UND STÄRKUNG DES IMMUNSYSTEMS
PROCEDES ET COMPOSITIONS POUR LA THERAPIE ET LA POTENTIALISATION GENETIQUES DE L'IMMUNITE ANTITUMORALE

(30) Priority: 14.09.1990 US 582711
(43) Date of publication of application: 21.07.1993
(73) Proprietor: THE JOHNS HOPKINS UNIVERSITY, Baltimore, MD 21205 (US); BOARD OF REGENTS, THE UNIVERSITY OF TEXAS SYSTEM, Austin, Texas 78701 (US)
(72) Inventor: FROST, Philip, Morris Township, NJ 07960 (US); VOGELSTEIN, Bert, Baltimore, MD 21208 (US); CHERNAJOVSKY, Yuti, London NW4 1AJ (GB); FEARON, Eric, R., Branford, CT 06405 (US); PARDOLL, Drew, Baltimore, MD 21210 (US)
(74) Representative: Wichmann, Hendrik, Dr.
(86) International application number: PCT/US1991/006612
(87) International publication number: WO 1992/005262

(56) References cited:
- PROCEEDINGS OF THE NATL. ACADEMY OF SCIENCES USA, vol. 86, December 1989, Washington, DC (US); Y. WATANABE et al., pp. 9456-9460
- IMMUNOLOGY TODAY, vol. 11, no. 6, June 1990, Elsevier Science Publishers Ltd., GB; S.J. RUSSELL, pp. 196-197
- CANCER RESEARCH, vol. 46, no. 9, September 1986, Philadelphia, PA (US); I.H. MAXWELL et al., pp. 4660-4664
- CANCER RESEARCH, vol. 48, no. 11, 01 June 1988, Philadelphia, PA (US); E.R. FEARON et al., pp. 2975-2980
- CHEMICAL ABSTRACTS, vol. 112, no. 19, 07 May 1990, Columbus, OH (US); F.L. MOOLTEN et al., p. 36, no. 171876a
- BIOLOGICAL ABSTRACTS, vol. 90, no. 5, 1990, AB-637; S. OSTRAND-ROSENBERG et al., no. 52893
- CELL, vol. 60, 09 February 1990, Cell Press, Cambridge, MA (US); E.R. FEARON et al., pp. 397-403

## Description

This invention relates generally to the field of immunotherapy of cancer, and more specifically, to preparation of tumor cell lines containing an exogenous gene encoding a polypeptide that potentiates the immune response to the tumor, for example, interleukin-2. The invention includes methods for cancer therapy by immunization with the immunopotentiating tumor cell.

The invention also provides methodology whereby one can selectively ablate in vitro and in vivo genetically altered tumor cells. A preferred embodiment of this aspect of the invention includes a tumor cell containing a first exogenous gene encoding an immunopotentiating polypeptide and a second exogenous gene encoding a "lethal" or "suicide" polypeptide, preferably under the control of an inducible promoter. Introduction of the second exogenous gene confers the ability to selectively kill the tumor cell by inducing a promoter in operative linkage to the gene encoding the lethal polypeptide to initiate transcription of the polypeptide. Diphtheria toxin or the thymidine kinase of Herpes simplex virus are exemplary lethal genes. An exemplary promoter employed is the 6-16 promoter, which is inducible with low levels of interferon. Methods for using the cells in cancer therapy are also provided.

Active immunotherapy is considered to be a promising approach to the treatment and particularly to the prevention of recurrences of human cancer. Specific active immunotherapy, one of the most promising approaches under investigation, involves activation of the host immune response against the tumor by immunization with tumor cells (which may be altered by mutagenesis, by treatment with a hapten, or by expression of foreign proteins) in order to activate specific effector cells of the immune system, such as cytolytic T lymphocytes. Nonspecific active immunotherapy may utilize microbial or chemical immunomodulators to activate natural killer (NK) cells, macrophages or lymphokine activated killing (LAK). Unfortunately, much of the promise of these approaches remains unfulfilled.

One of the most critical questions in cancer immunology is why the immune system fails to eliminate tumors. In the 1970's, Hewitt articulated the notion that most tumors did not express any tumor-specific or neoantigens and thus could not be recognized as "foreign" by the immune system. Indeed, virtually no tumor cell surface antigens recognized by antibodies were found to be tumor specific, and furthermore, most spontaneous murine tumors were considered "poorly immunogenic" as defined by their failure to be eliminated when transferred into syngeneic hosts (Hewitt et al., 1976). However, these same tumors could be rendered "immunogenic" by mutagenesis (Van Pel and Boon, 1982) when new antigens are expressed on the tumor cell surface.

It is possible that the immune system fails to eliminate tumors not because neoantigens are absent, but rather because the response to these neoantigens is inadequate. Therefore, a method for enhancing immunogenicity of the tumor cells so as to potentiate the host's immune response would provide a key advance in immunotherapy.

Failure to respond to tumor neoantigens may be due, at least in part, to a failure of T cell help. The molecular basis for Th function is the local secretion of lymphokines, such as interleukin-2 (IL-2), that act upon CTLs whose T cell receptors have first been engaged by the appropriate antigen-MHC complex (reviewed in Moller, 1980). The cytotoxic potential of NK and LAK cells is also enhanced by IL-2 (Grimm et al., 1982; Phillips and Lanier, 1986; Ortaldo et al., 1986). Although potentiation of tumor immunity by systemic injection of interleukin 2 has been attempted, those studies were hampered by the toxicity of the systemically administered IL-2. Therefore, a method for potentiating immunity to tumors by providing accessory T cell help in the location of the tumor is a more attractive option, which has long been needed for cancer therapy.

An additional difficulty in immunotherapy results from the problems inherent in administration of a living neoplastic cell to a patient. In the past, tumor cells used for immunization were treated prior to immunization to reduce their proliferative potential, e.g., by irradiation or treatment with mitomycin C. Unfortunately, either one of these methods of inhibiting replication also significantly diminishes the immunogenicity of the cells. It has been shown that mutagen induced variants that were irradiated with 8-10,000 Rads are no longer immunogenic (Sella, 1989; Boon, 1985). Similarly, murine tumor cells secreting IL-2 of IFN-γ lose their immune potential after irradiation. In addition, attempts at using membrane preparations of tumor cells also fail to produce convincing evidence of an immune response. It would therefore be advantageous to develop a means for using viable immunogenic tumor cells that could be deleted after they have induced an immune response.

The present invention provides for a novel immunopotentiating tumor cell variant, capable of inducing a host immune response to tumor antigens, comprising a selected immunopotentiating gene, for example, interleukin-2 and an exogenous lethal gene system.

In a first general embodiment, the invention provides a cellular preparation for potentiating the immune response to a tumor by a vertebrate organism, such as man or other mammal, birds, or fish. The preparation comprises cells, derived from the tumor, that contain an exogenous gene (e.g., one introduced by insertion of genetic material from outside the cell, for example, by transfection and selection of a stably transfected cell line) which encodes an immunopotentiating interleukin. In a preferred embodiment, the interleukin is interleukin 2. Although applicants are not bound by theory, evidence suggests that the interleukin 2 gene confers upon the cell the ability to induce a specific immune response, probably mediated primarily by T lymphocytes.

In a related embodiment, the invention also provides a composition for enhancing non-specific resistance to a tumor, that embodiment including a preparation of tumor cells containing an exogenous gene encoding interferon, preferably, interferon γ.

Although it is not necessary for practice of the invention, various gene combinations may produce greatly advantageous or even synergistic effects on potentiation of immunity to the tumor. Therefore, the invention also provides immunotherapeutic tumor cells that may contain more than one exogenous gene. For example, the cells may contain either an interleukin gene or an interferon gene or both and another gene encoding an yet another immunopotentiating polypeptide.

The immunopotentiating polypeptide may be defined as a polypeptide that enhances responsiveness of a host immune system to a tumor present in the animal. At least two categories of immunopotentiating genes can be used in practice of the invention. A first category, "immunopotentiating antigens," includes genes encoding a variety of antigens alien to the animal, such as virus envelope proteins, bacterial cell antigens, and other antigens, capable of eliciting an immune response in an animal immunized with a cell expressing such antigen. That category of genes is referred to here as "immunopotentiating antigens." Preferred species among the category include, for example, viral envelope proteins, e.g., the influenza hemagglutinin (HA) gene, the influenza neuraminidase gene, vaccinia genes, or vesicular stomatitis virus genes. Suitable bacterial antigens include, for example, the 65kDa antigen of M. tuberculosis and other bacterial antigens. Other immunopotentiating genes may include those encoding histocompatibility antigens allogenic to the host and other alloantigens.

A second category of immunopotentiating genes includes genes encoding proteins that may not be immunogenic to the host but nevertheless potentiate immunity by activating or enhancing the activity of cells of the immune system, such as T lymphocytes, natural killer cells, or lymphokine activated killer cells. Included in this category of immunopotentiating genes are those encoding a number of the lymphokines classified as "interleukins," and in particular, interleukin-2, -4, -5,-6,-1, or -3. Also included are in this category, although not necessarily working according to the same mechanism, are proteins known as interferons, and in particular interferon gamma.

The genes may be obtained by techniques known in molecular biology and introduced to a selected target cell (which is usually derived from the tumor) by transfection or by transformation with a vector suitable for use with the particular target cell. Particular techniques described by Sambrook, et al., Molecular Cloning, A Laboratory Manual, Second Edition, Cold Spring Harbor Press, 1989 (incorporated herein by reference) may facilitate practice of certain aspects of the invention.

In one embodiment of the invention, one may employ a population of selected target tumor cells containing at least one gene encoding an immunopotentiating antigen and a second immunopotentiating gene selected from the second category of immunopotentiating genes discussed above.

The invention also includes methods for potentiating the immune response of a vertebrate organism to a tumor by introducing into the organism a viable preparation of the tumor cells described here. In a preferred embodiment, the tumor cells are administered after reduction of tumor burden, e.g., by surgical resection, irradiation, or other appropriate techniques.

Of course, in many situations, it may be desirable to selectively ablate or destroy the administered tumor cells once they have achieved the desired function. Therefore, the tumor cells of the invention containing the immunopotentiating gene also contain a "lethal gene" that allows one to selectively kill the cell containing it should it become desirable to do so. In a preferred embodiment, the lethal gene will be coupled to a specifically inducible promoter, so that by treatment with the inducer, one can specifically promote transcription of the mRNA encoding the "lethal protein," which, in turn, is capable of directly or indirectly killing the host cell, for example, by inhibition of intracellular metabolism.

Therefore the invention provides a tumor cell, which comprises a first exogenous gene encoding a selected polypeptide and a second exogenous gene ("lethal gene") including a promoter, preferably a selectively inducible promoter, in operative linkage to DNA encoding a second selected polypeptide. The second selected polypeptide, a "lethal polypeptide," is capable of killing the tumor cell, for example, upon induction of a selectively inducible promoter or upon addition of a substrate for the lethal polypeptide, which substrate is converted by the lethal polypeptide to a molecule that is toxic for the tumor cell. By the term "operative linkage," it is meant that the promoter is located in a position relative to that of the DNA encoding the lethal polypeptide that allows the promoter to effectively direct transcription of the structural gene for the lethal polypeptide.

In a more specific embodiment directed to cancer immunotherapy, the invention provides a composition for potentiating the immune response to a tumor, the composition comprising a cell derived from the tumor, which cell contains a first exogenous gene encoding an immunopotentiating polypeptide and a second exogenous gene including a promoter in operative linkage to DNA encoding a polypeptide capable of killing the cell. Preferably, the promoter will comprise a specifically inducible promoter and the lethal polypeptide kills the cell upon induction of the promoter.

Although any of a number of suitable promoters, such as the SV40, cytomegalovirus, or actin promotors may find utility in practice of this aspect of the invention, the inducible 6-16 interferon α/β promoter described below is particularly advantageous. Alternatively, when a gene encoding a polypeptide which requires addition of an exogenous substrate for intracellular toxicity is used (such as the Herpes simplex thymidine kinase that requires gancyclovir), a constitutive promoter such as the Herpes simplex promoter may be employed.

A number of suitable lethal genes may be used. Suitable examples include the Herpes simplex virus thymidine kinase gene and the gene encoding Diphtheria toxin A chain.

The tumor cells containing immunopotentiating and lethal genes may also include yet a third exogenous gene, encoding a second immunopotentiating polypeptide. In a preferred embodiment, the third exogenous gene encodes an immunopotentiating antigen if the first immunopotentiating polypeptide is a cytokine and encodes a cytokine if the first immunopotentiating polypeptide is an antigen.

The invention also includes methods for producing the novel tumor cell derivatives. The invention includes, for example, a method for producing a tumor cell for therapy of a selected disease comprising introducing a first exogenous gene encoding a selected polypeptide into a tumor cell and introducing a second exogenous gene containing a promoter, preferably selectively inducibte promoter in operative linkage with DNA encoding a polypeptide capable of killing the tumor cell, into the tumor cell to produce a tumor cell having both the first and the second exogenous gene. Provision of a selectively inducible promoter allows one to kill the tumor cell upon induction of the promoter. Tumor cells, genes, and promoters, and lethal genes as described above are suitable for use with this aspect of the invention as well.

Of course, the invention also includes methods for use in gene therapy, and specifically, immunotherapy of cancer. An exemplary method for gene therapy of a vertebrate organism comprises introducing a first exogenous gene encoding a selected polypeptide into a tumor cell and introducing a second exogenous gene containing a promoter in operative linkage with DNA encoding a polypeptide capable of killing the cell into the cell to produce a cell having both the first and the second exogenous genes stably maintained in the cell; and administering the cell, preferably by injection into a the organism. In the preferred embodiment where the promoter is a selectively inducible promoter, the additional step of inducing the promoter to promote synthesis of the lethal polypeptide may be performed when warranted. Alternatively, when a constitutive promoter is used, e.g, in conjunction with the Herpes simplex thymidine kinase gene, administration of a second toxic agent, such as gancyclovir, may comprise an additional step of the methods. Of course, the Herpes simplex thymidine kinase gene may also be placed under control of a selectively inducible promoter, in which case both induction of the promoter and administration of the second agent may occur.

The methods may be used for immunotherapy of a variety of tumors, including, for example, melanoma, breast cancer, sarcoma, colon cancer, and ovarian cancer.

These and other aspects of the invention will become more apparent from a description of particular embodiments when read in conjunction with the drawings.

### FIGURE 1

Induction of CTLs after In Vivo Injection of CT26 cells and IL-2-Transfected CT26-IL-2⁺ Cells. Cells (1 x 10⁶) of either the CT26 or CT26-IL-2⁺ lines were injected subcutaneously into the left flank of BALB/c mice. After 2 weeks, splenocytes were removed and cultured 5 days with mitomycin C-treated CT26 cells in the presence of IL-2. At the end of culture, live cells were mixed with ⁵¹Cr-labeled CT26 targets at different effector to target ratios in a 4 hr ⁵¹Cr release assay. (a) CTLs generated from CT26 vs. CT26-IL-2⁺ cells. (b) Anti-CD4 and anti-CD8 blocking of CT26 lysis by splenocytes from mice immunized with CT26-IL-2⁺ cells. (c) Anti-MHC class I and class II blocking of CT26 lysis by splenocytes from mice immunized with CT26-IL-21⁺ cells.

### FIGURE 2

Protective Immunity against CT26 Cells Induced by Injection of CT26-IL-2⁺ Cells. BALB/c mice were injected subcutaneously in the left flank with 1 x 10⁶ CT26-IL-2⁺ cells followed by subcutaneous challenge in the right flank with 1 x 10⁵ CT26 cells either 2 weeks or 4 weeks later. Also shown is CT26 growth without CT26-IL-2⁺ preimmunization. Tumor growth was assessed every week by palpation and measurement. The pooled results of 20 mice per group are shown. Data is presented as a Kaplan-Meier plot.

### FIGURE 3

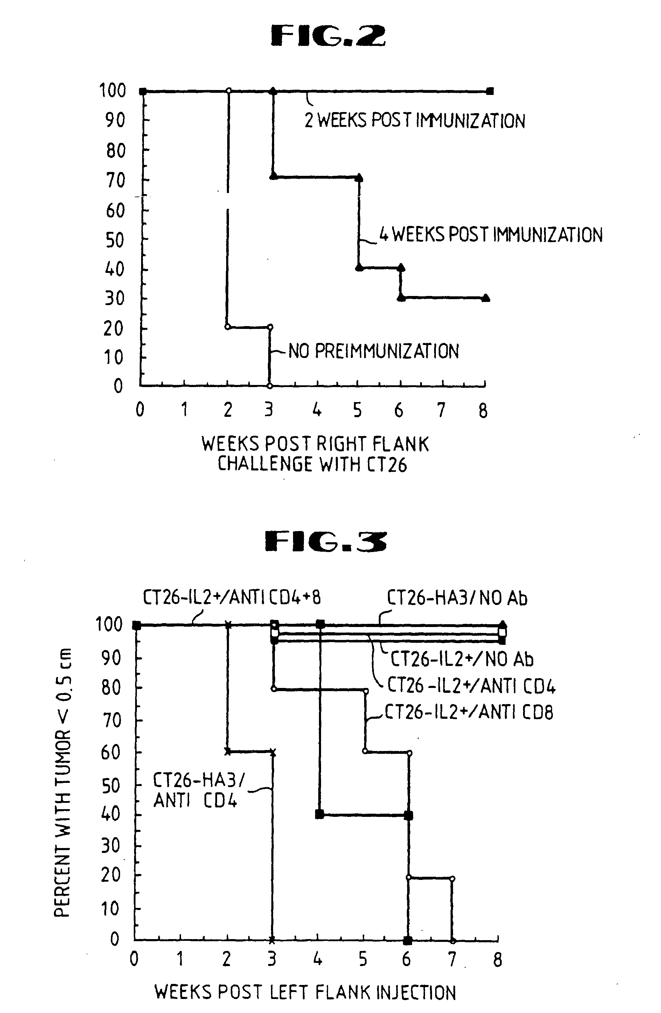

Effects of T Cell Subset Depletion on the In Vivo Response to CT26-IL-2⁺ and CT26-HA⁺ Cells. BALB/c mice were depleted in vivo of either CD4⁺, CD8⁺, or both CD4⁺ and CD8⁺ T cells by intraperitoneal injection of purified anti-CD4 or anti-CD8. They were then injected subcutaneously in the left flank with 1 x 10⁶ CT26-neo-IL-2⁺ cells. Also shown is the growth in normal and CD4-depleted BALB/c mice of a CT26-HA⁺ line rendered immunogenic by transfection with the influenza hemagglutinin gene. Tumor growth was measured every week and is presented as in Figure 2. Specific depletion of T cell subsets was documented as described in Experimental Procedures.

### FIGURE 4

Fluorescence intensity distribution based on the binding of labeled goat anti-mouse immunoglobulin to SP1 or CT-26 cells pre-treated with mouse anti-H2K^{k} or H2K^{d}, respectively.

### FIGURE 5

SP1 cells were treated with 10 U/ml of IFN_{-γ} for 24 hours. The cells were washed and a sample was removed for FACS analysis. The cells were returned to in vitro culture without additional IFN_{-γ} and samples were removed for FACS analysis at 48, 72 and 96 hours. This figure shows the fluorescence intensity distribution based on binding of goat anti-mouse immunoglobulin to IFN_{-γ} treated SP1 cells pre-treated with mouse anti-H2K^{k} antibody.

### FIGURE 6

Three distinct clones of SP1 cells transfected with the murine IFN_{-γ} gene were assessed for H2K^{k} expression. The figure demonstrates the fluorescence intensity distribution based on the binding of goat anti-mouse immunoglobulin to cells pre-treated with mouse anti-H2K^{k}.

### FIGURE 7

A group of six nude mice were injected s.c. with 1 x 10⁵ 6L cells. Two of the six resulting tumors were removed, cultured in vitro and assessed for IFN_{-γ} production and H2K^{k} expression. The individual tumors 6L-A and 6L-B were then injected into syngeneic mice and assessed for their tumorigenicity.

The following examples illustrate selected aspects of the invention but are not intended to limit it except as specified in the claims.

### EXAMPLE I (Comparative Example)

### Enhancement of Anti-Tumor Immunity by Immunization with Cells Transfected with an Exogenous Gene Encoding Interleukin 2.

The following example describes results obtained upon practice of one aspect of the invention potentiation of an anti-tumor immune response by administering cells transfected with a gene encoding an exogenous immunopotentiating cytokine, in this case, interleukin 2.

### A. Experimental Procedures

### 1. Cells

CT26 cells were obtained from M. Brattain (Brattain et al., 1980). The F10 subline of B16 melanoma cells (Fidler, 1975) was obtained from the NIH DCT tumor repository. RENCA is a murine renal cell carcinoma originally described by Murphy and Haushesky (1973). SS-5 is a spontaneous mammary adenocanthoma induced by methylcholanthene in one of our laboratories (P.F.). YAC-1, an MHC⁻ NK target cell line (Kiessling et al., 1975), was kindly provided by J. Wagner, Johns Hopkins University.

### 2. Transfections

DNA was introduced into cells as a coprecipitate with calcium phosphate (Graham and van der Eb, 1973; Wigler et al., 1979). The CT26-IL-2⁺ cell line was obtained by transfection with 5 µg of the plasmid vector pBCMG-neo-mIL-2, a bovine papilloma virus expression vector containing a murine IL-2 cDNA clone under the transcriptional control of a cytomegalovirus promoter with a rabbit β-globin intron, splice, and poly(A) addition signals; it also contains the Th5 neomycin-resistance gene (Karasuyama and Melchers, 1988; Karasuyama et al., 1989). Cells were exposed to the precipitate for 14-16 hr, washed once with Hanks' balanced salt solution without Ca²⁺ or Mg²⁺, refed with Dulbecco's modified Eagle's medium with 10% FCS, and incubated at 37°C. Selection in G418 at 400 µg/ml was begun 48 hr after cells were exposed to precipitate. The CT26-neo-IL-2⁻ line was produced by transfection of CT26 cells with a plasmid derived from pBCMG-neo-IL-2 by removing the cytomegalovirus early promoter, rabbit β-globin intron, and mIL-2 sequences. The hemagglutinin-expressing CT26-HA⁺ cell line (described in Fearon et al., 1988) was produced by cotransfection of CT26 with 5 µg of the plasmid vector pBV1-MTHA and pSV2-neo followed by G418 selection. FACS 3, clone 5 was used in the studies here. The B16-IL-2⁺ transfectant was generated similarly to the CT26-IL-2⁺ transfectant but was cloned by limiting dilution after G418 selection.

### 3. IL-2 Assays

Supernatants of transfected cells were assayed for IL-2 as previously described (Janis et al., 1989) by transferring dilutions to 96-well microtiter plates containing 3000 CTLL-2 cells per well. After 24 hr, [³H]-thymidine was added for 12 hr, after which incorporation was assessed with a PHD cell harvester. Units per milliliter IL-2 was calculated as the reciprocal of the supernatant dilution giving half-maximal proliferation of CTLL-2.

### 4. CTL Assays

For CTL assays, spleens were removed from BALB/c mice 2 weeks after subcutaneous injection in the left flank of 1 x 10⁶ CT26 or CT26-IL-2⁺ cells. Mitomycin C treatment of CT26 cells was performed by incubating them in 50 µg/ml mitomycin C for 45 min at 37°C followed by three washes with RPMI-10 FCS. In vitro stimulations were performed in 24-well plates for 5 days with 2 x 10⁵ CT26 stimulators and 6 x 10⁶ splenocyte responders per well plus 20-50 U/ml recombinant murine IL-2. ⁵¹Cr release assays were performed by mixing various numbers of effector cells with 5000 ⁵¹Cr-labeled targets per well in 96-well V-bottom plates. After 4 hr at 37°C, 100 µl per well was removed and counted in a gamma counter. Percent specific lysis ([cpmₑₓₚ - cpmₘᵢₙ]/[cpmₘₐₓ - cpmₘᵢₙ] x 100) is plotted on the y-axis for various effector to target ratios. CTL assays for the B16 melanoma system were performed in a similar fashion, except that incubations with ⁵¹Cr-labeled B16 targets were performed for 8 hr. For antibody blocking, a 1:100 dilution of ammonium sulfate-purified preparations of the following antibodies were added to microwells at the start of ⁵¹Cr release assays: GK1,5, monoclonal antibody (MAb) to CD4 (Dialynis et al., 19830; 2.43, MAb to CD8.2 (Sarmiento et al., 1980); M5-114, MAb to I-A^{d} + 1-E^{d} (Battacharaya et al., 1981); 28-14-8, MAb to L^{d} (Ozato et al., 1980); 35-1-2, MAb to K^{d} + D^{d} (Ozato et al., 1982). All blocking antibodies were carefully titered such that the concentrations used were 5-10 times the minimal concentration that yielded saturation binding to splenic lymphocytes as assayed by flow cytometric analysis of serially diluted antibody. Prior to use, blocking specificity was assessed as follows: anti-CD4 and anti-MHC class II antibodies inhibited in vitro secondary PPD-proliferative responses by more than 80% and allo-CTL lysis (B6 anti-BALB/c) by more than 5%; anti-CD8 and anti-MHC class I antibodies inhibited secondary PPD-proliferative responses by more than 5% and allo-CTL lysis by more than 80%.

### 5. In Vivo Antibody Depletions

In vivo antibody depletions were started 1-2 days prior to injection of the tumor. MAb GK1.5 was used for CD4 depletions and MAb 2.43 was used for CD8 depletions. Ammonium sulfate-purified ascites fluid (titered at >1:2000 by staining of thymocytes on the FACS) was injected intraperitoneally (0.1 ml per mouse) every other day for the first 3 weeks and then once per week afterward. Depletion of T cell subsets was assessed on the day of tumor injections, 3 weeks, and 5 weeks after tumor injection by flow cytometric analysis of lymph node cells stained with 2.43 or GK1.5, followed by fluorescein isothiocyanate-labeled goat antibody to rat IgG. For each time point of analysis, >99% depletion of the appropriate subset was achieved with normal levels of the opposite subset present (in the case of the single depletions).

### B. Results

### 1. Production of IL-2 Transfected CT26 Cells

The N-nitroso-N-methylurethane-induced murine colon tumor line CT26 chosen for study is poorly immunogenic; a small number of cells (1 x 10³-1 x 10⁴) injected into syngeneic (BALB/c) mice cause a lethal tumor and do not induce detectable tumor-specific CTLs (Fearon et al., 1988). CT26 cells were transfected with a bovine papilloma virus (BPV) vector containing a neomycin-resistance gene and a murine IL-2 CDNA. Transfectants were selected in the neomycin analog G418, and a G418-resistant line (CT26-IL-2⁺) prepared from more than 50 pooled clones of approximately equal size was chosen for further study.

To assay for IL-2 production, the CT26-IL-2⁺ line was plated at 5 x 10⁴ cells per well in a 24-well plate, and after 3 days, supernatants (1.5 ml per well) were removed and transferred in serial dilutions to the IL-2-dependent CTLL-2 cell line. They were found to contain 40 U of IL-2 activity (1 U defined as induction of 50% maximal CTLL stimulation in a 24 hr assay), indicating that CT26-IL-2⁺ cells secreted significant quantities of IL-2. No detectable IL-2 activity was found in supernatants from either the parental CT26 cells or CT26 cells transfected with the BCMG vector that had the IL-2 cDNA insert cut out.

### 2. CTL Generation Induced by CT26-IL-2⁺ Cells

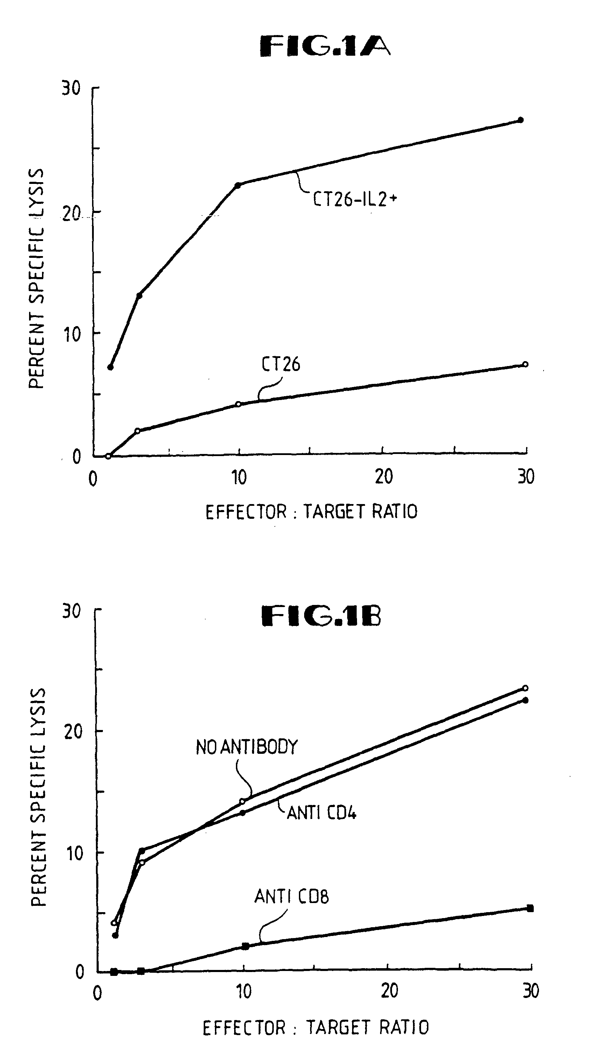

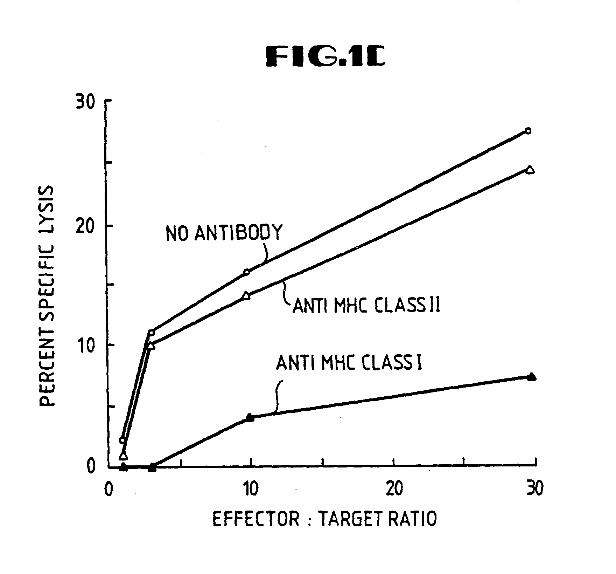

Subcutaneous injection of CT26 cells has been shown previously to elicit little if any detectable systemic CTL activity, even after a secondary in vitro stimulation in the presence of IL-2 (Fearon et al., 1988). However, after subcutaneous injection of the IL-2-producing CT26-IL-2⁺ cells, significant anti-CT26 CTL activity was detected in the spleen after secondary in vitro stimulation (Figure 1a). The majority of in vitro CTL activity was blocked by antibodies to CD8 and to MHC class I, but not by antibodies to CD4 or to MHC class II (Figures 1b and 1c). This suggested that CT26-IL-2⁺ cells indeed activated endogenous, MHC class I-restricted CD8⁺ CTLs. Virtually no cytolysis was observed against another BALB/c-derived tumor target (SS-5) or the MHC class I⁻ NK target, YAC-1 (<5% specific lysis at an effector to target ration of 100:1). These results demonstrate that most of the effector cells induced by CT26-IL-2⁺ immunization were antigen- and MHC class I-specific and that NK and LAK cells represent a minority of the activity measured in vitro.

### 3. In Vivo Immune Response Induced by CT26-IL-2⁺ Cells

We next sought to determine whether the CTL response elicited by CT26-IL-2⁺ cells correlated with in vivo immunity. Following even the largest subcutaneous injection of up to 1 x 10⁶ CT26-IL-2⁺ cells, no tumors were seen up to 8 weeks after injection. In contrast, tumors were present in all animals following injection of the parental CT26 cells by 2 weeks (Table 1). Thus, BALB/c mice were capable of rejecting 3-4 orders of magnitude greater numbers of CT26-IL-2⁺ cells than the parental CT26 cells. To demonstrate that rejection of CT26-IL-2⁺ cells is due to activation of CT26-specific effector cells by the locally produced IL-2, CT26 cells were transfected with the entire BPV vector sequences alone with the IL-2 insert removed. These transfectants (CT26-neo-IL-2⁻), which were confirmed not to secrete IL-2 by CTLL functional assay, produced tumors in BALB/c mice at a rate indistinguishable from the nontransfected CT26 cells (Table 1). In addition, we observed that injection of 1 x 10⁶ CT26-IL-2⁺ cells on the left flank did not inhibit the growth of 1 x 10⁵ CT26 cells on the opposite flank. Thus, the effect of IL-2 production by CT26-IL-2⁺ cells appeared initially to stimulate a local, rather than systemic, immune response.

Given the result that immunization with CT26-IL-2⁺ cells induced systemic CTLs after two weeks, as measured in vitro, we sought to determine whether this correlated with an in vivo antitumor response against the parental CT26 cells. Indeed, injection of CT26-IL-2⁺ cells completely protected mice against a challenge with 1 x 10⁵ CT26 cells 2 weeks later (Figure 2). This protection was not long-lived, as roughly 50% of mice challenged 4 weeks after immunization developed tumors (Figure 2). The protection was tumor specific in that other BALB/c tumors (SS-5, RENCA) grew normally when injected 2 weeks after CT26-IL-2⁺ immunization (data not shown).

### 4. CT26-IL-2⁺ Cells Bypass CD4⁺ T Helper Function

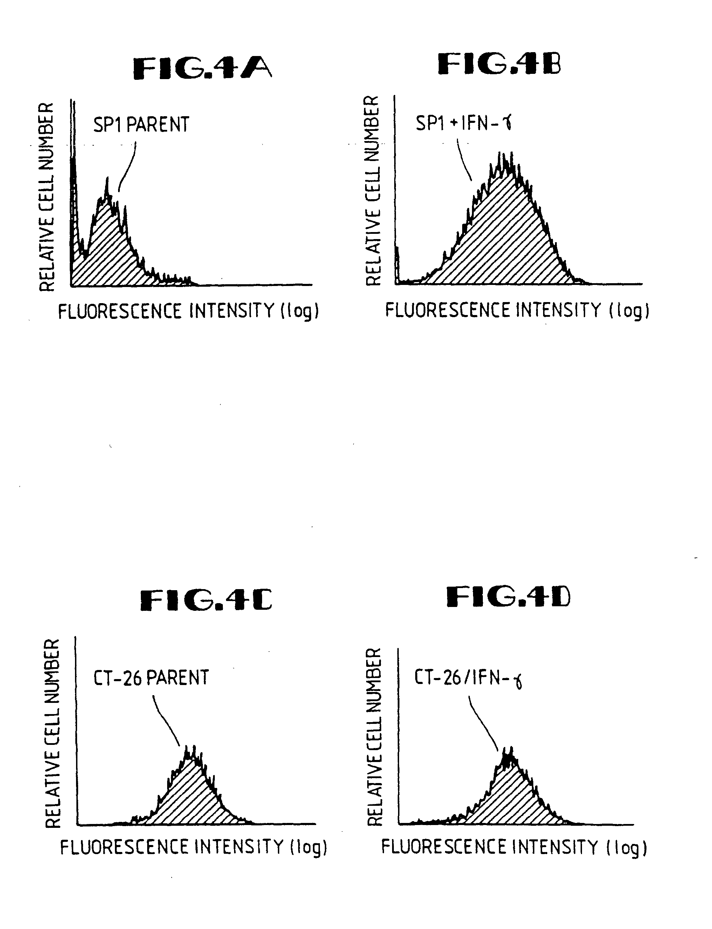

Because most T cell helper function is performed by the CD4⁺8⁻ subset of T lymphocytes and most MHC-restricted CTL function is performed by the CD4⁻8⁺ subset, we next investigated the effect of selective in vivo depletion of these subsets on the rejection of CT26-IL-2⁺ cells. Figure 3 shows that mice depleted of CD4⁺8⁻ T cells were fully capable of rejecting CT26-IL-2⁺ cells. Previous studies have shown that CT26 cells could be rendered immunogenic by transfection with a foreign gene such as influenza hemagglutinin (CT26-HA⁺; Fearon et al., 1988). The "failure of help" hypothesis suggested that the enhanced immune response induced by CT26-HA⁺ cells might be mediated, at least in part, by CD4⁺ helper T cells responding to MHC class II-restricted epitopes on the exogenously introduced hemagglutinin gene product. In support of this hypothesis is the observation that CT26-HA⁺ cells failed to be rejected by CD4-depleted mice (Figure 3). Taken together, these results support the concept that CT26-IL-2⁺ immunization effectively bypasses Th function in the generation of an antitumor CTL response.

### 5. CD8⁺ Cells Are Required In Vivo For Tumor Rejection

Mice depleted of CD4-8⁺ cells were incapable of rejecting CT26-IL-2⁺ cells, indicating that this T cell subset is involved in the antitumor response in vivo (Figure 3). It is noteworthy, however, that there was a significant lag in the tumor's growth kinetics relative to the kinetics of CT26 growth in normal BALB/c mice. While this partial response could be due to the action of residual CD4⁻8⁺ cells, none were detected in lymph nodes by flow cytometric analysis either at the time of initial tumor injection or during the course of in vivo antibody treatment. A similar delay in tumor growth was seen in the presence of both CD4 and CD8 depletion. It is therefore likely that an IL-2-responsive, CD4⁻8⁻ effector cell population was additionally involved in the in vivo antitumor response. Three IL-2-responsive candidates are NK cells, LAK cells, and T cells bearing the γδ T cell receptor (reviewed in Pardoll et al., 1987; Brenner et al., 1988; Raulet, 1989). As most of the cells in these populations are CD4⁻8⁻, they would still be present in CD8-depleted mice. Other potential effector cells involved in the antitumor response, based on histologic analysis of regressing tumors, include macrophages and mast cells (data not shown).

### 6. CTL Generation and In Vivo Immunity Induced by IL-2-Producing Melanoma Cells

To assess whether the induction of systemic immunity was a general feature of tumors engineered to secrete IL-2, we transfected a second poorly immunogenic tumor, B16 melanoma, with the BCMG-IL-2 vector. The B16 melanoma is a highly aggressive melanocyte tumor of C57BL/6 origin. After transfection, G418-resistant clones were chosen (B16-IL-2⁺) that produced quantities of bioactive IL-2 equal to or greater than cells, when assayed under identical conditions on CTLL cells. Table 2 shows the results of a representative clone. While small numbers of B16 melanoma cells caused tumors in syngeneic C57BL/6 mice, B16-IL-2⁺ cells were completely rejected. As with the CT26 tumor, greater CTL activity was generated against the parental B16 tumor in mice injected with B16-IL-2⁺ cells. C57BU6 mice injected with B16-IL-2⁺ cells developed protective immunity in vivo against challenge with B16 cells in a dose-dependent manner. The protection was not as great as seen in the CT26 system, as roughly half the mice immunized with 1 x 10⁶ B16-IL-2⁺ cells did eventually develop tumors after challenge with 1 x 10⁵ parental B16 cells (Table 2).

It is noteworthy that MHC class I expression on B16 melanoma cells is extremely low - roughly 5- to 10-fold lower than on CT26 cells (data not shown). This may account for the apparently lower efficiency of protection against challenge with parental tumor in the B16 than in the CT26 system, although the faster growth rate of B16 melanoma cells may be an additional contributing factor.

In addition to the colon tumor and melanoma data presented here, similar results have been recently observed with an IL-2-transfected murine CBA-SP1 sarcoma and the rat Dunning prostate carcinoma (data not shown). The fact that similar effects were seen with two tumors of widely differing cellular origins suggests that the principles outlined here may be generalizable to a variety of cancers. In other studies, however, a spontaneous mammary adenocarcinoma derived from CBA mice was transfected with the IL-2 gene and used to immunize CBA mice against growth of the parental tumor. In those studies, the IL-2⁺ transfectant did not induce a significant immunity against the parental tumor.

We have also prepared a number of additional transfectants expressing HA and/or secreting IL-2 or IFN-γ. It is believed that the dually transfected cells should improve the immunogenic status of the tumor.

### EXAMPLE II (Comparative Example)

### Production of Tumor Cells Transfected with an Exogenous Gene Encoding Gamma Interferon and Demonstration of Enhanced Non-Specific Tumor Resistance

The following example describes yet another aspect of the invention, transfection of tumor cells with a gene encoding a cytokine capable of non-specifically enhancing tumor resistance, probably through a mechanism dependent on cells other than T lymphocytes. In the following example, the non-specific enhancement of tumor resistance was produced by injecting mice with a tumor cell transfected with the gene encoding gamma interferon.

More specifically, SP1 murine adenocarcinoma cells transfected with the murine IFN_{-γ} gene expressed IFN_{-γ} (SP1/IFN_{-γ}), failed to grow in syngeneic hosts, and grew in nude mice. The rejection of IFN_{-γ} cells was related to the amount of IFN_{-γ} produced and appeared to be mediated primarily by nonspecific cellular mechanisms, though some role for T cells in the afferent arm of this response is possible. SP1 are H2K^{k} negative but express class I antigens when producing IFN_{-γ}. However, class I MHC expression, while likely necessary, was insufficient in itself to prevent tumor growth since secretion of >64 U/ml IFN_{-γ} was needed to inhibit tumorigenicity while only 8 U/ml IFN_{-γ} could induce class I antigens. Similar results were obtained with the murine colon carcinoma CT-26, a tumor that constitutively expresses class I MHC antigens, further supporting the contention that Class I MHC expression is not essential for the rejection response induced by IFN_{-γ}. The failure of SP1/IFN_{-γ} cells to protect against a challenge with parent SP1 cells argues that factors other than IFN_{-γ} production or class I MHC expression are needed to induce a protective response against weakly or non-immunogenic tumor cells.

### A. Materials and Methods

### 1. Mice.

CBA and BALB/c female mice between 6 and 8 weeks of age were obtained from the Frederick Animal Facility of the National Cancer Institute.

### 2. Tumors.

The origin of the SP1 spontaneous mammary adenocarcinoma has been described previously (Frost et al., 1987). SP1 grows readily in syngeneic mice after s.c. inoculation and is poorly, if at all, immunogenic and expresses little if any class I MHC antigen. The CT-26 colon adenocarcinoma was obtained from M. Brattain (Brattain et al., 1980). MDW1 is an immunogenic variant of the MDAY-D2 lymphoma (Kerbel, 1979).

### 3. Culture Conditions.

Cells were cultured in GIBCO RPMI 1640 medium supplemented with 50,000 U of penicillin and streptomycin, 150 mg of L-glutamine, 20 mM 4-(2-hydroxyethyl)-1-piperazine ethanesulfonic acid (HEPES), 375 mg of sodium bicarbonate, and 10% fetal calf serum (Irvine Scientific, Santa Ana, CA). All tumors were tested periodically for the presence of *Mycoplasma* using the Gen Probe RNA hybridization method (Gen Probe, San Diego, CA); contamination with *Mycoplasma* was never observed. In addition, the cells were tested for murine antibody production (Microbiological Associates, Bethesda, MD) and found to be free of 13 murine pathogenic viruses.

### 4. Antisera.

The monoclonal antibodies used in these studies were as follows: Murine anti-D^{k}/K^{d} (clone 15-5-5S) was kindly provided by Dr. B.E. Elliott (Queen's University, Kingston, Ontario, Canada). Murine anti-K^{d} (clone 16-1-11N) was purchased from the Litton Company (Charles, SC). Murine anti-K^{k}/D^{k} Ab (clone H 100-27/55), anti-I-A^{k} (clone 14V18), and anti-D^{k} were purchased from the Cedarlane Company (Ontario, Canada). Murine anti-I-A^{d} antisera was obtained from Becton Dickinson (San Jose, CA).

### 5. In Vitro IFN-_{γ} Treatment.

Recombinant murine IFN_{-γ} was obtained from Genentech, Inc. (San Francisco, CA). 3 x 10⁵ tumor cells were plated in five 10-cm plastic dishes and incubated with 1-100 U/ml of IFN_{-γ} for 4 days. On the fourth day, IFN_{-γ}-treated cells were aliquoted and reincubated with 1-100 U/ml of IFN_{-γ} for an additional 3 d. The cells were then analyzed for the expression of class I or class II MHC antigens.

### 6. Flow Cytofluorometry.

Quantitative analysis of MHC expression on cell surfaces were performed using FACS. Cells were scraped from tissue culture plates using a rubber policeman and incubated, with murine anti D^{k}, D^{d} or IA^{k} or IA^{d} antibodies for 30 min at 4°C. After being washed, the cells were incubated with FITC-conjugated goat anti-mouse IgG antibodies at 4°C for 30 min, fixed with 1% paraformaldehyde and examined within one week using cytofluorometry.

### 7. Transfection of Cells With DNA.

DNA was introduced into SP1 or CT-26 cells either as a coprecipitate with calcium phosphate (Graham and van der Eb, 1973) or via use of the lipofectin reagent (Felgner et al., 1987). The SP1-neo and CT-26/neo cell lines were obtained by transfection with a mixture of 1 ug of the plasmid vector pSV2neo with 1 ug of mouse liver DNA. The SP1-IFN_{-γ} lines were obtained by transfecting SP1 cells with 1 ug of pSV2neo and 10 ug of pGEM3-SVMu IFN_{-γ}. The latter is a pGEM vector containing an SV40 promoter and the murine IFN_{-γ} gene and was kindly provided by Dr. R. Suzuki of the Department of Immunology at the M.D. Anderson Cancer Center. After selection in geneticin, the colonies form individual plates were pooled and assessed for IFN_{-γ} production.

### 8. Anti-Viral Assay for Interferon Activity.

The method of Mory et al., was used (Mory et al., 1981) to assay IFN activity. This assay relies on the inhibition of vesicular stomatitis virus growth in murine LTK cells. Control wells were treated with recombinant IFN_{-γ} or medium alone. Supernatants from the experimental cell cultures were added at doubling dilutions and their ability to inhibit VSV viral growth assessed by determining how many wells contained viable LTK cells.

### 9. Assessment of Cell-Mediated Cytotoxicity.

Cytotoxicity was measured using the previously described ¹¹¹In-release assay (Wiltrout et al., 1978).

### 10. Separation of Spleen Cells.

Nylon wool adherent (NWA) and nonadherent cells (NWNA) were obtained by the method described in Julius et al., (1973).

### B. Results

Prior to the transfection studies, we ascertained whether the SP1 cells would express MHC class I antigens in response to IFN_{-γ}. In addition, we determined if the addition of exogenous IFN_{-γ} had a cytostatic effect on either the SP1 or CT-26 cell lines. One to 1000 U of IFN_{-γ} were added to semiconfluent cultures of SP1 or CT-26 cells for four days. Class I MHC expression was then measured and cell viability and replication was measured each day for five days and compared to non-IFN_{-γ}-treated cells. CP1 cells grew normally in as much as 100 IFN_{-γ}; but the growth of CT-26 cells was inhibited by 17% in 1000 U/ml IFN_{-γ}. The growth rates of SP1 cells transfected and secreting 256 U/ml IFN_{-γ} (6L) were the same as that of the parent SP1 cells. Similarly, CT-26 cells producing 64 U/ml IFN_{-γ} had the same growth rate as parent CT-26 cells.

Figure 4 demonstrates the expression of H2K^{k} in Sp1 cells as well as H2K^{d} in CT-26 cells as assessed by FACS using specific murine monoclonal antibodies. Fewer than 6-7% Sp1 cells expressed H2K^{k}, but all expressed IA^{k} (data not shown). In contrast CT-26 cells expressed both class I and II MHC antigens (Class II data not shown). After treatment of SP1 cells with exogenous IFN_{-γ} the expression of class II antigens did not change but a dramatic shift of the FACS curve to the right was readily detectable with anti K^{k} antibodies. IFN_{-γ} treatment had no effect on the constitutively expressed class I or II antigens of CT-26 cells.

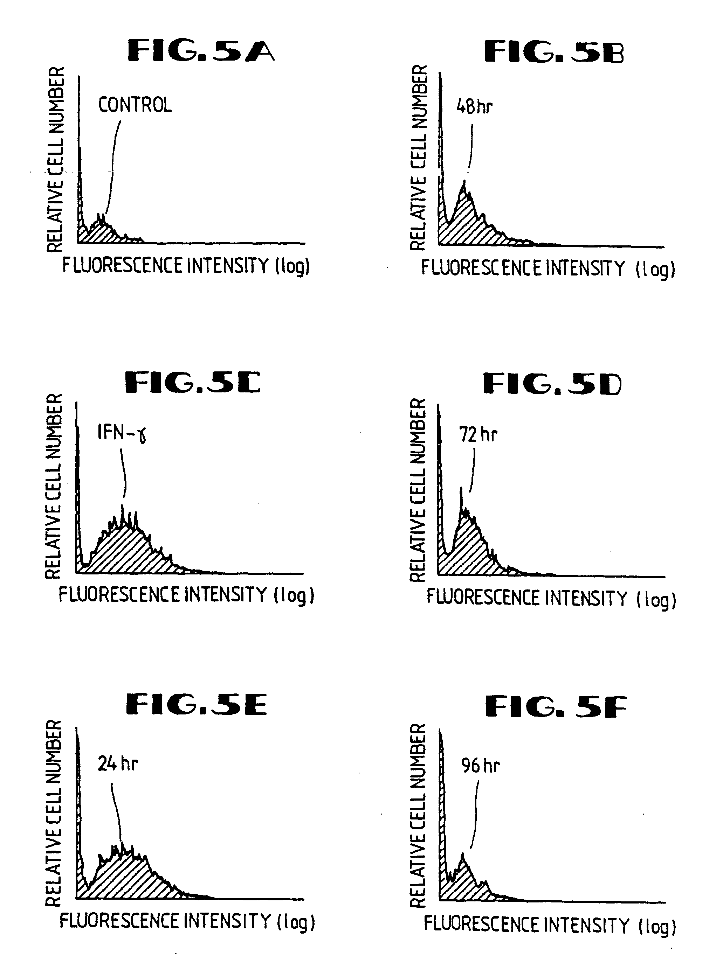

We next determined the duration of the effect of exogenous IFN_{-γ} on class I expression by SP1 cells. These experiments served as a basis for the use of transfection with the IFN_{-γ} gene as a means for providing sustained production of IFN_{-γ} and expression of class I MHC antigens. SP1 cells were treated with 100 u/ml of IFN_{-γ} for three days. The cells were washed and either assessed for H2K^{k} expression or recultured. This was repeated at 24, 48, 72 and 96 hours after the removal of IFN_{-γ} from the medium. Figure 5 shows that the H2K^{k} expression returned to baseline levels within 48 hours after the removal of IFN_{-γ} from the medium. It is reasonable to assume that a similar reduction in H2 expression would occur if IFN_{-γ}-treated cells were injected in vivo.

### 1. Effect of IFN_{-γ} Transfection on Class I MHC Expression.

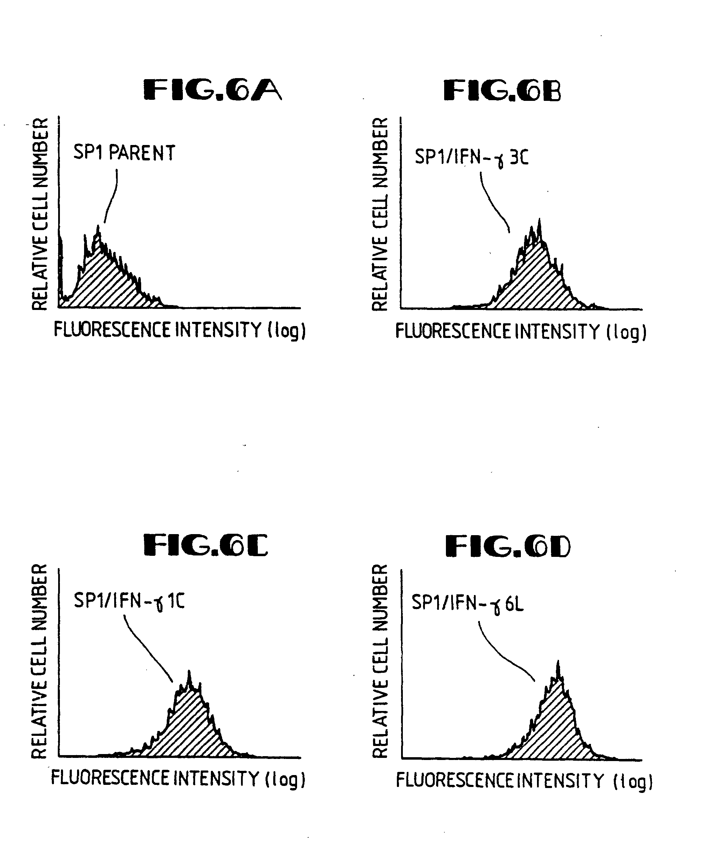

After transfection, both the SP1 and CT-26 lines were tested for the secretion of IFN_{-γ}. Three SP1-IFN_{-γ} cell populations--1C, 3C and 6L-- were selected for study based on the amount of IFN_{-γ} secreted. The results are shown in Table 3. The CT-26/IFN_{-γ} cells were similarly shown to produce IFN_{-γ}, but to a lesser degree than the SP1/IFN_{-γ} cells (8 U/ml vs 256 U/ml). Of interest is the demonstration in all three SP1/IFN_{-γ} cell lines of an equivalent increase in MHC class I expression (Figure 6, the results for the CT-26 cells are shown below)). In addition, all three lines expressed class II MHC antigens (IA^{k}) at a level comparable to the nontransfected parent cells (data not shown). SP1 cells transfected with the neo^{R} gene alone did not express class I MHC antigens.

### 2. Effect of IFN_{-γ} Gene Transfection on Tumor Growth In Vivo.

IFN_{-γ} were injected s.c. into CBA mice. Table 3 summarizes the data from several such experiments. These cell lines were selected because they all demonstrated a significant level of H2K^{k} expression (Figure 6), when compared to five additional populations that were screened and found not to express H2K^{k} and were thus presumed not to be producing IFN_{-γ}. 1C and 3C grew at challenge doses of 1 x 10⁵. In contrast, 6L cells failed to grow in 14 of 15 animals injected with 1 x 10⁶ cells; the parent SP1 cell line grew when as few as 10³ cells were injected s.c. Immunogenicity correlated with the level of IFN_{-γ} production rather than H2K^{k} expression (Table 3 and Figure 6) since all three lines expressed equivalent levels of MHC antigen but only the 1C and 3C cell lines grew vigorously. 6L cells did grow in nude mice. Sp1 cells transfected with the neo^{R} gene alone grew in a manner identical to nontransfected SP1 cells (Kerbel et al., 1987).

### 3. Nonspecific Cytotoxic Response to 6L.

Table 4 shows that viable 6L cells induced a cytotoxic response reactive with itself and the parent SP1 tumors. This response was mediated by nylon wool adherent (NWA) spleen cells but not by nylon wool non-adherent (NWNA) cells. The cytotoxicity of the NWA spleen cells against both SP1 and 6L was always greater than that produced by the whole spleen cell population. In addition, NWA cells were reactive (though to a lesser degree) against the unrelated MDW1 tumor cell line. These results implied that a non-specific mechanism was responsible for the rejection of 6L cells. This view was supported by our failure to diminish the splenic cytotoxic response after depletion of immune spleen cells with anti-CD4, anti-CD8 and anti-CD3 antibodies plus complement (data not shown).

To further assess the nonspecific nature of the rejection of 6L cells, 1 x 10⁶ 6L cells were mixed with 1 x 10⁵ SP1 cells and injected together into the left flank of CBA mice. Five of 10 mice developed tumors at 5 weeks. Syngeneic CBA mice injected with 1 x 10⁴ SP1 cells develop tumors within 2 weeks. The five tumors that grew were removed and placed in tissue culture, allowed to grow for two days, and then exposed to geneticin. In all cases, the cells placed in culture failed to grow in the presence of geneticin, indicating that these were parent SP1 cells, which are sensitive to 500 ug/ml geneticin (6L cells carry the neo^{R} originally used in the co-transfection with the IFN_{-γ} gene). The fact that the growth of 6L mixed with SP1 cells was markedly impaired argues that nonspecific mechanisms induced by the 6L cells were responsible in part for its failure to grow in vivo.

This conclusion was indirectly supported by experiments in which immunization of CBA mice with 6L failed to protect animals against a significant challenge with parental SP1 cells (Table 5). Fifty percent of immunized animals were protected against a challenge with 1 x 10⁴ cells injected in the opposite flank. However, as few as 5 x 10⁴ SP1 cells induced tumors in 100% of the immunized animals. In addition, the injection of animals simultaneously in the left flank with 6L and right flank with SP1 had no effect on the growth of the SP1 cells, though the 6L cells failed to grow.

### 4. Role of Class I MHC Antigen Expression.

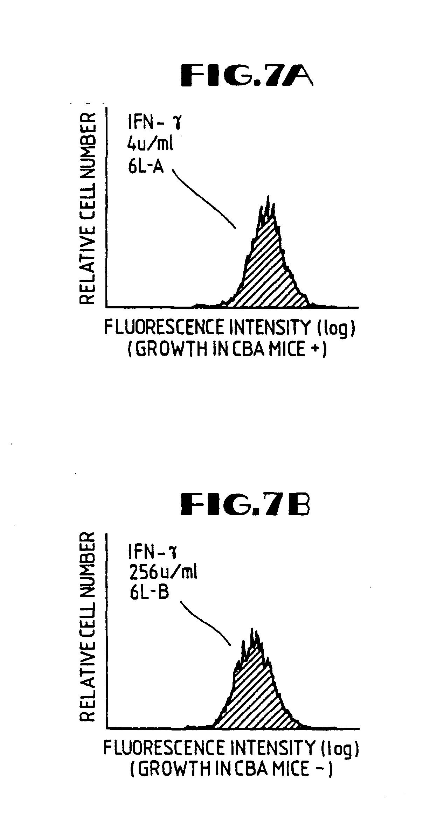

It remained to be determined whether the effect of IFN_{-γ} production was strictly due to its enhancement of class I MHC expression or if IFN_{-γ} produced locally had other effects on the host. 6L cells were injected into 6 nude mice and produced tumors. Two of these tumors were removed (6L-A and 6L-B) and recultured. These cell lines were then assessed for IFN_{-γ} production and H2 expression. Figure 7 shows that 6L-A and 6L-B had equivalent expression of H2K^{k} even though 6L-B produced 64x more IFN_{-γ}. The reinjection of 6L-A and 6L-B into groups of six CBA mice demonstrated that low IFN_{-γ}-producing 6L-A cells grew in all animals despite expressing H2K^{k}. In contrast, high IFN_{-γ} producing 6L-B cells failed to grow.

### 5. The CT-26 Tumor.

We next determined the effect of IFN_{-γ} expression of murine tumor cells that constitutively express class I MHC antigens. CT-26 murine adenocarcinoma cells were transfected with the pGEM-SVMu-IFN_{-γ} plasmid and the original pool of transfected cells was found to secrete 8 U/ml of IFN_{-γ}. Table 6 shows that at doses of 1 x 10⁵ s.c. or 5 x 10⁴ i.v., CT-26/IFN_{-γ} cells eventually grew, but considerably more slowly than parent CT-26 cells.

The failure of CT-26/IFN_{-γ} cells to grow in vivo was directly related to their IFN_{-γ} production and could not be explained by any direct cytopathic effect of IFN_{-γ} because 8 U/ml IFN_{-γ} had no effect on CT-26 growth in vitro. In addition to reduced growth in vivo, CT-26/IFN_{-γ} induced a cytotoxic response detectable in the spleens of immunized mice (data not shown). Parent CT-26 cells have never been shown to engender any detectable cytotoxic response.

### EXAMPLE III

### Production of Immunopotentiating Tumor Cells Co-Transfected With an Exogenous Gene Encoding a Selected Immunopotentiating Polypeptide and a Second Exogenous Gene Encoding a Polypeptide Capable of Ablating the Host Cell on Specific Induction.

The following example describes yet another aspect of the present invention, the production of an immunogenic tumor cell that may be selectively ablated in circumstances when it becomes desirable to do so. In this aspect of the invention, cells are transfected with a selected gene encoding a desired polypeptide, e.g., the immunopotentiating polypeptide, and a second gene that encodes a polypeptide capable of ablating (killing) the host cell. The second gene, referred to as the "ablation gene" or the "lethal gene," is preferably under the control of a specifically inducible promoter. Thus, when it is desired to eliminate some or all of the transfected cells from the host (for example, at about 14 days after administration of the transfected tumor cells), one may initiate such elimination by directly or indirectly activating the inducible promoter to stimulate the synthesis of the lethal polypeptide, for example, by administering to the host an amount of the inducer sufficient to initiate transcription of the polypeptide. Specific examples of embodiments of this aspect of the invention are described below.

### A. Selection and transfection of genes encoding immunopotentiating polypeptides

Selection of the most advantageous immunopotentiating polypeptide for use in a given situation will likely depend on a number of parameters, including, for example, tumor type, location, and immune status of the patient. Genes encoding such polypeptides are either currently available, or may be obtained using present techniques in molecular biology, for example, by using techniques described by Sambrook et al., in Molecular Cloning, A Laboratory Manual, Cold Spring Harbor Press, 1989 (which is incorporated herein by reference). Transfection of the target cell type may generally be carried out essentially according to the protocol set forth in example I above. In the examples discussed more specifically below, the genes selected for transfection are those encoding the immunopotentiating polypeptides, IL-2, interferon gamma, and influenza hemagglutinin.

### B. Selection and transfection with a specifically inducible lethal gene system.

### 1. Selection of a suitable promoter

Although a number of promoters, e.g., cytomegalovirus, SV40, or actin and the like could conceivably be used in accordance with the invention, we have selected the interferon-inducible 6-16 promoter for use in a preferred embodiment. Use of this promoter has a number of advantages because the levels of IFN in normal blood are very low. Therefore, the injection of IFN can trigger this gene in a very specific manner.

The human IFN-regulated gene 6-16 is tightly controlled by IFN α/β. The 0.9 Kb cDNA of 6-16 encodes for a 13 kilodalton protein which contains a signal peptide sequence at the amino terminal end; however, neither the function or the subcellular localization of the protein (secreted or membrane bound) has been defined (Kelly, 1996). Untreated cells have no detectable levels of 6-16 mRNA in their cytoplasm or nuclei. 6-16 mRNA synthesis can be detected as early as 5 minutes following the interferon alpha treatment; its synthesis in the nucleus of the cell peaks at three hours and decreases to basal levels within 8-12 hours (Friedman, 1984, Kelly, 1986). However, the mRNA of 6-16 is post-transcriptionally regulated and its presence in the cytoplasm can be detected even 12 hours after the nuclear synthesis has ceased (Friedman, 1984). The tight regulation of the 6-16 promoter has been used as a tool to obtain IFN-resistant mutants by linking the 6-16 promoter to selectable marker genes that also allow for back selection in case of low level deregulated expression (Pellegrini, 1989).

We have cloned and characterized the promoter region of 6-16 (Porter, 1988), its cis-acting IFN-regulatory sequences and the trans-acting factors with which they interact by 5' deletion analysis, transfection experiments, DNase-footprinting, gel retardation, in vitro transcription, and linking the promoter to reporter genes (Dale, 1989, Porter, 1988), (Chernajovsky, 1989).

The 6-16 promoter contains a direct repeat of 40 nucleotides (from -168 to -89) which includes the IFN-regulatory sequence that can confer IFN inducibility to heterologous promoters (Chernajovsky, 1989, Porter, 1988). This region binds a nuclear protein(s) of a molecular weight of 55 kilodaltons and can form in vitro a DNA-protein complex which is IFN modulated (Chernajovsky, 1990).

Another cis-acting element located at -450 binds constitutively to a nuclear protein of 80 kilodaltons and acts to down regulate expression of this promoter in conjunction with the direct repeat. By itself, this element seems not able to exert any transcriptional function.

In addition, the promoter of 6-16 has a CCAAT box located in the non coding strand of the promoter and a TATA box (Porter, 1988)(Chernajovsky, 1989)(Chernajovsky, 1990).

### 2. Selection of Lethal Genes

Lethal genes that may be used in accordance with the invention include genes encoding a variety of proteins that inhibit or arrest intracellular metabolism, and in particular, those leading to inhibition of key cellular functions such as DNA synthesis, transcription and translation, or those that paralyze other necessary cellular functions. Exemplary lethal genes that may prove useful in accordance with the invention include for example, the genes encoding ricin beta chain, pertussis toxin, etc. As discussed below, selection of a particular lethal gene will often be governed by the physiologic characteristics of the cell to which it will be introduced.

For some rapidly growing tumors or cells, an agent that will specifically inhibit DNA synthesis would offer an advantage. However, for others, e.g., solid tumors that grow slowly and do not proliferate very actively, such a DNA synthesis inhibitor may be less useful. In such cases, an agent that can specifically block another metabolic function of the cell, such as protein translation, could prove more relevant. Examples of lethal gene systems designed to address each of those cell types are discussed below.

For DNA synthesis inhibition, a preferred gene encodes the enzyme thymidine kinase from Herpes simplex virus. That enzyme is able to actively phosphorylate the thymidine analog gancyclovir (GANC) (1-(-deoxy-2-fluoro-β-D-arabinofuranosyl) -5-ethyluracyl), which after conversion to the 5' triphosphate by cellular kinases, inhibits DNA polymerase (Mansour, 1988, Mansuri, 1987). The basis for the discriminating effect of GANC on cells expressing HSV-tk and the cellular tk enzyme, is the fact that the substrate requirements of the HSV-tk protein are less stringent than those of the cellular tk. Thus, cells expressing HSV-tk are sensitive to the toxic effects of GANC; normal cells are spared.

For protein synthesis inhibition, a preferred gene is that encoding the α chain of Diphtheria toxin (DT-A). DT-A ADP-ribosylates elongation factor 2, producing protein synthesis inhibition and cell death. This gene has been used successfully in transgenic mice to specifically ablate pancreatic acinar cells when linked to the enhancer/promoter of the elastase I gene (Palmiter, 1987).

### 3. Construction of Selectively Inducible Lethal Gene Constructs

The following prophetic examples describe construction of genetic constructs suitable for use in transfection of selected tumor cells in accordance with the invention.

### a. Construction of a Herpes Simplex virus tk gene driven by the 6-16 promoter

A Bgl II-Pvu II (1775 bp) fragment from the plasmid pAGO containing all of the coding and polyadenylation signals of the tk gene (with 57 nucleotides of 5' untranslated region) is isolated and cloned together with the Xhol -HpaII (2. 3Kb) fragment of the 6-16 promoter, which spans all of the promoter region up to nucleotide - 4. The cloning is done in three steps. First, the BglII to Pvull fragment of the HSV tk is subcloned into the BamH1-Sma1 site of pUC18 to produce a plasmid named TK.pr⁻ . A diagnostic restriction carried with Smal and EcoRI should yield two fragments, one of 360 and another one of 4 Kb.

Then, the fragment Xho1 (preceded by the polylinker sites HindIII to Sal1 of pUC18) to Hpall of the 6-16 promoter is subcloned into the AccI to HindIII sites of pUC18 to produce plasmid 6-16-Xho1-HpaII. Finally, the promoter of 6-16 is cloned in front of the tk gene by isolation of the Hind III-XbaI (2.3 kb) fragment from the plasmid 6-16 XhoI-HpaII into the XbaI-HindIII site of TK.pr⁻ to yield plasmid 6-16 TK, which contains the tk gene under the 6-16 promoter control.

### b. Construction of a diphtheria toxin α gene driven by the 6-16 promoter

A similar approach to the cloning of the 6-16.TK is used to produce a DT-A gene driven by the 6-16 promoter. First, the 795 bp Bgl II fragment (flanked by EcoR1 and Hind III sites) is subcloned from the plasmid pDT-A (kindly provided by Dr. I Maxwell, University of Colorado). pDT-A contains the full coding sequence of the diphtheria α toxin gene. Because the toxin polypeptide is a polyprotein having both the α-(24 kilodaltons) and β (38 kilodaltons) chains (Maxwell, 1987, Leong, 1983), the DNA is modified as follows to express the α chain only.

The initiator methionine codon is substituted from GTG to ATG, and the first two amino acids are changed to Asp-Pro. In addition, the DNA fragment contains at the carboxy terminal end a synthetic Ser-Leu -stop codon from small SV40 t antigen with a splice signal but devoid of polyadenylation signal (Maxwell, 1986, Palmiter, 1987).

To clone the DT-A insert, the DT-A is digested with HindIII, and then ligated to a 940 bp HindIII-EcoR1 SV40 fragment containing the polyadenylation sequences of T antigen, the remainder of the insert is recut with EcoR1. Consequently, the insert contains EcoR1 sites at the ends, and the DT-A fragment has a polyadenylation signal 3' of it. This EcoR1 1.7 kb fragment is cloned into the EcoR1 site of the 6-16 promoter plasmid.

The orientation of the insert is assessed by restriction with BgIII. There are three BgIII sites, one at -603 in the 6-16 promoter and two at each end of the DT-A 795 bp fragment. When the insert is in the right orientation, BgIII digestion should release the 600 fragment of the 6-16 promoter till the BgIII of the DT-A insert and the 795 by DT-A insert and the rest of the plasmid. In the opposite orientation, the fragments released will be of 795 (DT-A fragment) and 1.5 kb (containing the 940 bp SV40 3' and polyadenylation sequences + the 603 bp of the 6-16 promoter and the rest of the plasmid.

### 4. Introduction of the Selected Genes Into the Target Cell

The selected immunopotentiating and lethal genes may be introduced into the cell by any of the methods known to those of skill in the art. However, in a preferred embodiment, cells will be transfected by the calcium phosphate coprecipitation method (Graham, 1973), lipofection, or electroporation. Tumor cells containing selected transfected immunopotentiating genes, e.g. HA or cytokines, may then be transfected with the lethal genes, or, conversely, cells can first be transfected with the lethal gene construct. Preferably, in addition to the lethal gene, the lethal gene construct will contain suitable marker and selection genes, which allow detection and selection of transfectants. Preferred among these are the marker plasmid pHC110 (Hall, 1983), containing the bacterial β-galactosidase gene, and the selectable marker pSV2hygro (Blochlinger, 1984). Cells are transfected with those markers at a molar ratio of 15:15:1 and selected in 400µg/ml hygromycin B. In addition, selected clones are sensitive to gancyclovir or IFN and stain positively with X-gal (Price, 1987, Thompson, 1989).

### 5. Preclinical Studies

Because the molecular mechanisms of toxicity of the diphtheria toxin and gancyclovir are different, a number of different assays may be performed in order to determine the concentrations of gancyclovir or IFN needed to obtain the cytotoxic response in different cell types. The biological assays described below will be performed.

### a. Assessment of concentration of Gancyclovir necessary for killing cells transfected with the Wild Type tk

The concentration of gancyclovir known to affect (by more than 90%) the plating efficiency of mouse embryonalstem cells containing one copy of the HSV tk gene, is in the range of 10⁻⁶ to 5x10⁻⁶ M. The concentration of gancyclovir needed to kill more than 90% of HSV-tk transfected tumor cells will be determined by plating efficiency assays and assays of [³H]-thymidine incorporation. The plating efficiency assay is conducted by plating 10⁴ cells in 10 cm² plates, with increasing concentrations of gancyclovir. Every three days the media is changed, and fresh gancyclovir added. After 9 days, the cells are stained with Giemsa, and the number of colonies per plate is determined. The assay of [³H]-thymidine incorporation is performed similarly to the assay described below for biological fluids but using the HSV-tk tumor transfected cells.

### b. Assessment of concentration of Gancyclovir and IFN necessary for killing cells transfected with the 6-16 tk ablation vector

The incorporation of GANC into DNA will probably depend on both its extracellular concentration and the intracellular concentration of the HSV tk enzyme. Cells having increased production of tk induced by addition of IFN may have enhanced sensitivity to GANC. This synergy is studied using increasing concentrations of both cytotoxic agents in conditions in which the concentration of one of them remains constant. Increased toxicity will be assessed both by [³H]-thymidine incorporation and plating efficiency assays.

### c. Determination of gancyclovir concentrations in biological fluids

In order to be able to rapidly assess the concentration of gancyclovir in biological samples such as serum, [³H]-thymidine incorporation assays are set up with LTK⁻ cells transfected with the HSV tk gene and selected in HAT media. The assay is carried out in the presence of aminopterin (1.8 ng/ml) and hypoxanthine (1.36 ng/ml) in order to avoid the synthesis of dTMP from dUMP by thymidine synthetase but to allow the synthesis of AMP and GMP through the HGPRT purine salvage pathway. Under these conditions the incorporation of [³H]-thymidine will depend mainly on the amount of inhibition of DNA synthesis produced by gancyclovir after its phosphorylation and processing to a triphosphate derivative that inhibits DNA polymerase, and the incorporation of [³H]-thymidine will be independent of the internal UMP/TMP pools. The fact that these cells do not contain an endogenous mammalian tk gene will be of great advantage because the cells will be extremely sensitive to gancyclovir.

The assay for DNA synthesis is performed as follows. Cells are seeded at 15-25 x 10³ cells per well in a 96-well microplate. The cells are incubated in triplicate with or without known concentrations of gancyclovir in the presence of hypoxanthine and aminopterin. After 24-28 hrs, cells are labeled with [3H]-thymidine 915 mCi/ml; 25 Ci/mmol; 1 Ci + 37 GBq; Amersham) for 1 hr. The cells are then washed twice with phosphate-buffered saline, treated with 5% trichloracetic acid for 30 min at 4°C, and washed three times with 5% trichloroacetic acid. The precipitate is dissolved in 0.1 ml of 0.2 M NaOH at 37°C for 30 min and neutralized by 0.01 ml of 2 M CH1; radioactivity is determined in a β-scintillation counter. The concentration of gancyclovir from animal fluids could be found by two fold dilutions of the samples and comparison of the extent of inhibition obtained to the inhibition curve of the standard used.

### d. Assessment of concentration of IFN necessary for inhibition of protein synthesis and killing of cells transfected with the 6-16 DT-A plasmid

To investigate the dose of IFN and time of exposure necessary to produce a cytotoxicity of more than 90%, inhibition of protein synthesis in cells transfected with the plasmid 6-16 DT-A is measured using a sensitive colorimetric assay.

Tumor cells cotransfected with the 6-16 DT-A plasmid, pSV2neo (for selection in G418) and with pCH110, which contains the bacterial β-galactosidase enzyme under the control of the early SV40 promoter, are selected by culture in G418. Resistant cell clones are isolated and tested by southern blotting to prove that all plasmids are present. Selected cell clones are those which are killed by IFN and have detectable levels of β-galactosidase activity.

The inhibition of protein synthesis due to the activation of the diphtheria toxin will produce a decrease in the amount of βgalactosidase that can be detected in a colorimetric assay using the substrate pNPG (p-nitrophenyl-β-D-galactopyranoside). This substrate of β-galactosidase turns yellow upon cleavage by the enzyme and the accumulation of the yellow product can be measured at a wavelength of 220 nm in Elisa plates. This enzymatic system will also be useful in vivo. The presence of cells containing the β-galactosidase gene can be visualized in histological samples by using the substrate X-gal that, upon cleavage, produces a blue precipitate in the cells. Therefore, this assay allows the determination of the presence of tumor cells containing lethal gene constructs in situ in animal tissue after treatment with IFN.

### 6. In vivo experiments

The following prophetic examples describe practice of the invention in a variety of murine tumor models.

CT-26 cells with or without immunopotentiating and/or lethal genes are injected into syngeneic BALB/c animals. Similarly, SP1 and RENCA cells described below are injected into their syngeneic hosts CBA and BALB/c mice, respectively. To estimate the immunogenic potential of the cells expressing the lethal genes, 1 x 10³ to 10⁷ cells will be injected in the right flank of the animals while the parental nontransfected cells will be injected on the left flank. The number of tumor cells required to kill in 50% of the animals will be estimated by the method of Reed and Muench (Reed, 1938) as previously described (Fearon, 1988).

### 7. Models

### a. H4A

The H4A cell line was derived by transfection of the SP1 mammary adenocarcinoma with the influenza virus hemagglutinin (HA) gene. Cells expressing HA were selected four times using Fluorescence Activated Cell Sorting (FACS) as previously described (Fearon, et al., 1989), and the H4A cell line was obtained. H4A fails to grow in syngeneic hosts at a challenge dose of 1 x 10⁴, but does grow if 1 x 10⁵ H4A cells are injected subcutaneously. Despite the growth of the cells, the animals do mount a cytotoxic T cell response, though this response is inadequate or occurs too late to offset tumor growth.

CBA mice will be injected with H4A, or H4A transfected with either HSV-tk or DTA. When the resultant tumor reaches different sizes (2-4 mm, 6-8 mm, 1 mm or 2 mm) all animals will be treated in the following manner to kill the injected cells.

| TUMOR CELL INJECTED | TREATMENT |
|---|---|
| H4A | None |
| H4A | GANC |
| H4A | IFN-α |
| H4A | GANC + IFN-α |
| H4A - HSV tk | None |
| H4A - HSV tk | GANC |
| H4A - HSV tk | IFN-α |
| H4A - HSV tk | GANC + IFN-α |
| H4A-DTA | None |
| H4A-DTA | GANC |
| H4A-DTA | IFN-a |
| H4A-DTA | GANC + IFN-a |

The initial doses of IFN-a (Hoffman-LaRoche, Nutley N.J.) will be 5 x 10⁴ units/injection s.c. three times a week for two consecutive weeks. GANC (Syntex, Palo Alto, CA) will be given s.c. at 20 mg/kg/day initially.

Animals in whom the primary tumor has been rejected are then:
a) be assessed for a splenic cytotoxic T cell response;
b) be challenged with 1 x 10⁴, 1 x 10⁵, or 5 x 10⁵ parent cells to detect immunity to a parent tumor challenge.

### b. CT-26/IFN-γ

The CT-26 murine colon carcinoma, when transfected with and expressing IFN-γ, becomes partially immunogenic. While a 1 x 10⁵ challenge dose of parental CT-26 cells is lethal to normal animals in 3 weeks; 60% of the CT-26 IFN-γ cell injected animals survive greater than 8 weeks. These cells appear to be immunogenic, but the response they engender is sometimes insufficient to prevent tumor growth. In addition, animals immunized with viable CT-26/IFN-γ were not protected against a parent CT-26 challenge. Furthermore, irradiated CT-26/IFN-γ cells were also nonprotective against a challenge with parental cells.

### c. RENCA Model

RENCA is a renal cell carcinoma of BALB/c mice. The injection of 5 x 10⁴ RENCA cells into the subrenal capsule of syngeneic mice results in the development of a u 1 cm tumor with two weeks. If the tumor bearing kidney is surgically removed, the animals will all develop extensive lung metastases within two weeks.

### C. Human Immunotherapy

Due to the necessary constraints imposed on human therapy, the present invention has not yet been tested in human subjects. However, it is contemplated that the invention may be used in humans having selected tumors, for example, of the colon, breast, ovary, melanoma, and sarcoma. Generally, it is preferred to select for treatment patients who have had a discrete primary tumor removed for which there is a high risk of recurrence. In a usual situation, tumor cells from the patient will be isolated and transfected with suitable immunopotentiating and lethal genes. Of course, it will be appreciated that when the immunopotentiating gene is a cytokine, such as interleukin-2, a gene encoding a human cytokine will be used. After transfection, the cells will be selected to obtain cell lines which are stably transfected with the selected genes.

In the ideal situation, the patient will first be treated with any of a number of regimes designed to reduce tumor burden, such as surgical resection, irradiation, etc., prior to administration of the tumor cell immunogen. The cells are then allowed to immunize the host for a selected period of time, usually about 14 days. Throughout this period of time, suitable assays may be performed to assess the immune status of the patient to the particular tumor in question. However, it should be realized that where the immunization is local in nature, systemic immunity may not be observed in all cases, and it may thus be necessary to obtain lymphocytes from the tumor site for such studies. In any event, after a selected period of time in which the host has been effectively immunized against its tumor cells, the lethal gene will be caused to eliminate the remaining immunizing tumor cells. In the instance when the selectably inducible promoter employed is a 6-16 promoter described here, alpha interferon will be administered to the patient in a dose effective to initiate transcription of the lethal gene but not so high as to be acutely toxic to the patient. The alpha interferon may also be administered locally to the site of the vaccine introduction. When the Herpes simplex thymidine kinase gene is used, it will be necessary to administer gancyclovir. When the thymidine kinase gene is driven by the 6-16 promoter, administration of both gancyclovir and interferon alpha will be warranted. While definite parameters for each of these administrations and the like have not yet been ascertained, they should be readily ascertainable by those of ordinary skill in the art with the assistance provided here.

The foregoing description of the invention has been directed to particular preferred embodiments in accordance with the requirements of the patent statutes and for purposes of explanation and illustration.

For example, numerous methods for introducing the exogenous genes into the target tumor cells may be used. In addition, various selected genes may be transfected in accordance with the invention. For example, certain aspects of the invention, such as the provision of a selectively inducible lethal gene, may not be limited to use in immunotherapy but may be more widely applicable to any situation in which it is desirable to be able to specifically ablate a genetically engineered cell in vivo. In addition, the transfected tumor cell lines of the present invention are useful for ex vivo immunization, for example, in activation of LAK cells for infusion into a patient according to the procedure described in U.S. Patent # 4,690,915, issued to Rosenberg, or in vitro activation of cytotoxic T lymphocytes or for promoting growth of cytokine dependent cell lines, such as IL-2 dependent cell lines. The ability to purge in vitro cultures of the transfected tumor cells by simply inducing transcription and translation of the lethal gene provides an additional advantage. Therefore, it is apparent that the invention may also be utilized, with any number of suitable modifications within the state of the art.

### REFERENCES

The following references may facilitate understanding or practice of certain aspects of the present invention. Inclusion of a reference in this list is not intended to and does not constitute an admission that such reference represents prior art with respect to the present invention.
Battacharya et al., J. Immunol. 127:2488-2495.
Biochlinger and Diggelman, Mol. Cell. Biol. 4:2929-2931 (1984).
Boon, Immunology Today 6:307-311 (1985).
Brattain, et al., Cancer Res. 40:2142-2146 (1980).
Brenner, et al., Adv. Immunol. 43:133-192 (1988).
Cantor and Boyse, J. Exp. Med. 141:1390-1399 (1975).
Chernajovsky et al., Lymphokine Res. 9:199-212 (1990).
Chernajovsky, FEBS. Lett. 258(2):323-30 (1989).
Colbere-Garapin et al., Proc. Natl. Acad. Sci. USA 76:3755-3759 (1979).
Dale et al., Proc. Natl. Acad. Sci. USA 86(4):1203-7 (1989).
Dialynas et al., J. Immunol. 131:2445-2452 (1983).
Fearon et al., Cancer Res. 48:2975-2980 (1988).
Fearon et al., Cell 60:397-403 (1990).
Felgner et al., Proc. Natl. Acad. Sci. 84:7413 (1987).
Fidler, Cancer Res. 35:218-234 (1975).
Friedman et al., Cell 38:745-755 (1984).
Frost and Chernajovsky, Transformation injury and the unicellular phenotype of malignant cells 9:93-98 (1990).
Frost et al., Cancer Res. 47:2690 (1987).
Graham and van der Eb, Virology 52:456-467 (1973).
Grimm et al., J. Exp. Med. 155:1823-1831 (1982).
Hall et al., J. Molecular and Applied Genetics 2:101-109 (1983).
Herberman, NK Cells and Other Natural Effector Cells, New York, Academic Press (1982).
Hewitt et al., Br. J. Cancer 33:241-259 (1976).
Hui et al., Nature 311:750-752 (1984).
Itaya et al., Caner Immunol. Immunother. 28:248-252 (1989).
Janis et al., Science 244:713-715 (1989).
Jenkins et al., Proc. Natl. Acad. Sci. USA 84:5409-5413 (1987a).
Jenkins et al., Immunol. Rev. 95:113-135 (1987b).
Julius et al., Eur. J. Immunol. 3:645 (1973).
Karasuyama and Melchers, Eur. J. Immunol. 18:97-104 (1988).
Karasuyama et al., J. Exp. Med. 169:13-25 (1989).
Keene and Forman, J. Exp. Med. 155:768-782 (1982).
Kelly et al., Embo. J. 5(7):1601-6 (1986).
Kerbel, Amer. J. Path. 97:609 (1979).
Kerbel et al., Proc. Natl. Acad. Sci. 84:1263 (1987).
Kiessling et al., Int. J. Cancer 15:933-940 (1975).
Leong et al., Science 220:515-517 (1983).
Lo et al., Cell 53:159-168 (1988).
Lurquin et al., Cell 58:293-303 (1989).
Mansour et al., Nature 336:348-352 (1988).
Mansuri et al., J. Med. Chem. 30:867-871 (1987).
Markman et al., Nature 336:476-479 (1988).
Maryanski et al., Nature 324:578-579 (1986).
Maxwell et al., Mol. Cell. Biol. 7:1576-1579 (1987).
Maxwell et al., Cancer Res. 46:4660-4664 (1986).
Mitchell, Proceedings of the Second Conference on Immunity to Cancer, Williamsburg, VA, Nov. 9-11, 1987; 288:323-336 (1989).
Möller, Immunol Rev. 51 (1980).
Moore et al., Cell 54:777-785 (1988).
Mory et al., Eur. J. Biochem. 120:197 (1981).
Mulligan and Berg, Science 209:1422-1427 (1980).
Murphy and Haushesky, J. Natl. Cancer Inst. 50:1013-1025 (1973).
Ortaldo et al., J. Exp. Med. 164:1193-1205 (1986).
Ozato et al., J. Immunol. 125:2473-2477 (1980).
Ozato et al., Transplantation 34:113-120 (1982).
Palmiter et al., Cell 50:435-443 (1987).
Pardoll et la., FASEB J. 1:103-109 (1987).
Pellegrini et al., Mol. Cell Biol. 9(11):4605-12 (1989).
Phillips and Lanier, J. Exp. Med. 164:814-825 (1986).
Porter et al., Embo J. 7(1):85-92 (1988).
Price et al., Proc. Nat. Acad. Sci. USA 84:156-160 (1987).
Raulet, Annu. Rev. Immunol. 7:175-208 (1989).
Reed and Muench, Am. J. Hyg. 27:493 (1938).
Sarmiento et al., J. Immunol. 125:2665-2672 (1980).
Sella et al., Clin. Exp. Metastasis 7:97-105 (1989).
Sitkovsky and Paul, Nature 322:306 (1988).
Smith et al., J. Bacteriol. 141:184-189 (1980).
Tanaka et al., Science 228:26-30 (1984).
Tepper et al., Cell 57:503-512 (1989).
Thompson et al., Cell 56:917-310 (1989).
Townsend et al., Cell 44:959-968 (1986).
Trenn et al., J. Immunol. 140:1101-1106 (1988).
Van Pel and Boon, Proc. Natl. Acad. Sci. USA 79:4718-4722 (1982).
vonBoaehmer and Haas, J. Exp. Med. 150:1134-1142 (1979).
Wallich et al., Nature 315-301-305 (1985).
Widmer et al., J. Exp. Med. 166:1447-1455 (1987).
Wigler et al., Proc. Natl. Acad. Sci. USA 76:1373-1376 (1979).
Wiltrout et al., J. Natl. Cancer Instit. 61:183 (1978).
Zinkernagel et al., J. Exp. Med. 147:897-911 (1978).

**Table 1**

| Growth of CT26, CT26-IL-2⁺, and CT26-neo-IL-2⁻ in BALB/c Mice | | |
|---|---|---|
| Tumor | Injection Dose^{a} | # Mice with Tumor/# Mice Injected |
| CT26 | 1 x 10³ cells | 9/10^{b} |
| | 1 x 10⁴ cells | 20/20^{c} |
| | 1 x 10⁵ cells | 25/25^{c} |
| | 1 x 10⁶ cells | 20/20^{c} |
| CT26-IL-2⁺ | 1 x 10⁴ cells | 0/20^{e} |
| | 1 x 10⁵ cells | 0/35^{e} |
| | 1 x 10⁶ cells | 2/55^{e} |
| | 1 x 10⁷ cells | 4/15^{e} |
| CT26-neo-IL-2⁻ | 1 x 10⁴ cells | 10/10^{b} |
| | 1 x 10⁵ cells | 20/20^{d} |
| | 1 x 10⁶ cells | 10/10^{d} |

| | | |
|---|---|---|
| ^{a} BALB/c mice were injected subcutaneously on the left hind leg, and tumor growth was observed. | | |
| ^{b} All mice had tumors by 3 weeks. | | |
| ^{c} All mice had tumors by 2 weeks. | | |
| ^{d} Most mice had tumors by 2 weeks, all had tumors by 3 weeks. | | |
| ^{e} Tumor-free mice were observed from 6-12 weeks. | | |

**Table 2**

| In Vivo Growth, Protective Immunity, and CTLs Generated by IL-2-Transfected B16 Melanoma Cells | | | | |
|---|---|---|---|---|
| | # Cells Injected^{b} | Tumor Growth^{c} | Protection against Challenge with B16^{d} | Lysis of B16^{e} |
| B16 | 1 x 10⁴ | 4/5 | --- | --- |
| | 1 x 10⁵ | 5/5 | --- | 16% |
| | 1 x 10⁶ | 5/5 | --- | --- |
| B16-IL-2⁺^{a} | 1 x 10⁴ | 0/9 | 9/9 | --- |
| | 1 x 10⁵ | 0/8 | 6/8 | 26% |
| | 1 x 10⁶ | 0/9 | 5/9 | --- |

| | | | | |
|---|---|---|---|---|
| ^{a} B16-IL-2⁺ transfectant was produced by transfection with pBCMG-neo-mIL-2 as described in Experimental Procedures. | | | | |
| ^{b} Cells were injected subcutaneously into the left flank as in Table 1. | | | | |
| ^{c} Tumor growth was measured weekly. Animals were observed for 7 weeks. All animals that developed tumors did so by 2 weeks after injection. | | | | |
| ^{d} Challenge with the 1 x 10⁵ parental B16 cells was done in the right flank 2 weeks after immunization with B16-IL-2⁺ cells as in Figure 3. Animals were observed for 7 weeks after challenge. Numbers represent animals that acquired tumors. These tumors were observed to arise between 3 and 5 weeks after challenge. Tumor-free animals were observed for 7 weeks. | | | | |
| ^{e} CTL assays were performed after a 5 day stimulation with mitomycin C-treated B16 cells as in Figure 1a. Numbers represent % specific lysis after an 8 hr ⁵¹Cr release assay at 100:1 effector to target ratio. | | | | |

**TABLE 3**

| Growth of IFN_{-γ} Transfected SP1 Tumor Cells | | | | |
|---|---|---|---|---|
| Tumor | IFN_{-γ} Production (Units/ml) | No. of mice with tumor/No. of mice challenged | | |
| | | 10⁵ | 5x10⁵ | 10⁶ |
| SP1 Parent SP1/IFN_{-γ} | --- | 10/10 | NT | NT |
| 1C | 16 | 2/3* | NT | NT |
| 3C | 32 | 3/3* | NT | NT |
| 6L | 256 | 0/15 | 0/10 | 1/15° |
| Groups of CBA mice were injected s.c. with either 10⁵, 5x10⁵ or 10⁶ cells from three different transfection plates (1C, 3C and 6L). The animals were then observed for tumor growth for 90 days. | | | | |

| | | | | |
|---|---|---|---|---|
| * Growth at 24 days | | | | |
| ° Growth at 90 days | | | | |

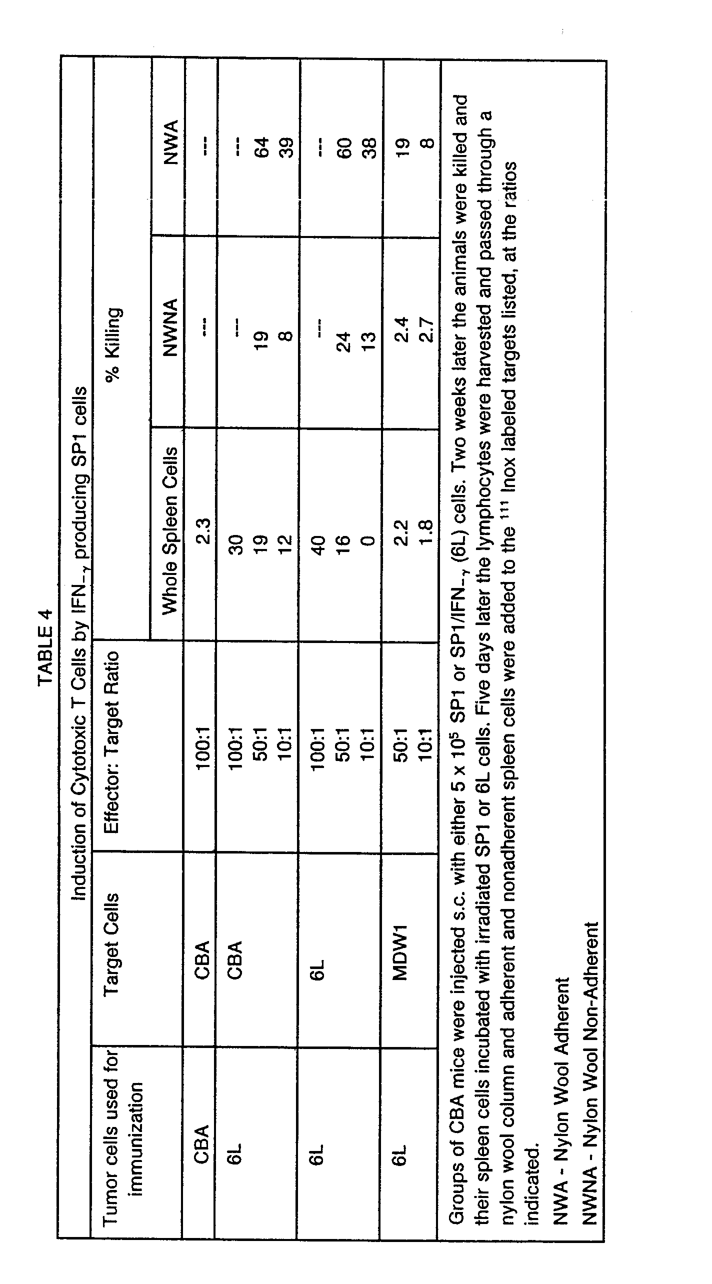

**TABLE 5**

| Immunoprotection by IFN-_{γ} transfected SP1 cells against a challenge with parent cells | | | | | |
|---|---|---|---|---|---|
| Tumor | Immunization dose | | Challenge Dose | | |
| | 1st | 2nd | 10⁴ | 5x10⁴ | 10⁵ |
| SP1 | --- | --- | 10/10* | 10/10 | 10/10 |
| SP1/IFN-_{γ} 6L | 2x10⁶ | 10⁶ | 9/18 | 10/10 | 18/19 |
| CBA mice were immunized with 10⁶ 6L cells on day 0 and again on day 14. On day 19, immunized and control animals were challenged with 10⁴ or 10⁵ parent SP1 cells. The animals were then observed for tumor growth. | | | | | |

| | | | | | |
|---|---|---|---|---|---|
| * Number of mice with tumors/Number of mice challenged. | | | | | |

**TABLE 6**

| Growth of IFN-_{γ} producing CT-26 tumor cells | | | | | |
|---|---|---|---|---|---|
| Tumor cells | Challenge dose | Route | Tumorigenicity | | |
| | | | 3w | 5w | 7w |
| CT-26 | 1x10⁵ | s.c. | 9/10* | --- | --- |
| CT-26/IFN-_{γ} | 1x10⁵ | s.c. | 2/15 | --- | 9/15 |
| CT-26 | 5x10⁴ | i.v. | --- | 5/5 | --- |
| CT-26/IFN-_{γ} | 5x10⁴ | i.v. | --- | 0/5 | 3/5 |
| Groups of BALB/c mice were injected s.c. or i.v. with CT-26 or CT-26IFN~_{γ} cells and observed for tumor growth and survival. | | | | | |

| | | | | | |
|---|---|---|---|---|---|
| * Number of mice with tumors/Number of mice challenged. | | | | | |

## Claims (Claims for the following Contracting State(s): AT, BE, CH, DE, DK, FR, GB, IT, LI, LU, NL, SE)

1. A composition comprising a tumor cell having been genetically altered by introducing into the cell at least one exogenous immunopotentiating gene, and an exogenous lethal gene including a promoter in operative linkage, for use in a method of stimulating in a subject a systemic immune response to a tumor.

2. The composition of claim 1, wherein said tumor is selected from melanoma, adenocarcinoma, and sarcoma.

3. The composition of claim 1 or 2, wherein the tumor cell is derived from a mammal, in particular a human.

4. The composition of any of the preceding claims, wherein the at least one immunopotentiating gene codes for a cytokine selected from interleukin (IL), in particular IL-4; and interferon, in particular gamma interferon; or codes for a foreign antigen selected from a viral protein, in particular the influenza hemagglutinin (HA); a bacterial cell antigen, particularly from *Mycobacterium tuberculosis* and more particularly the 65kD antigen of *M*. *tuberculosis*; and an allogeneic histocompatibility antigen.

5. The composition of any of the preceding claims, wherein said exogenous lethal gene is the gene for Diphtheria toxin A chain.

6. The composition of any of the preceding claims, wherein said promoter is an inducible promoter, particularly the 6-16 interferon promoter.

7. The composition of any of the preceding claims, wherein the exogenous immunopotentiating gene and/or the exogenous lethal gene has been introduced into the cell by transfection with a vector.

8. The composition of claim 7, wherein the transfection of the cell with the vector is mediated by calcium phosphate coprecipitation, liposomes or electroporation.

9. A tumor cell obtained by
(a) introducing at least one exogenous immunopotentiating gene, in particular as defined in claim 4, encoding a selected polypeptide into a tumor cell; and
(b) introducing an exogenous lethal gene as defined in any of claims 1, 5 or 6 into the tumor cell; to produce a tumor cell having the exogenous genes stably maintained in the cell;
for use in a method for gene therapy of cancer of a mammalian subject.

10. A method for producing a tumor cell for gene therapy of cancer comprising:
(a) introducing at least one exogenous immunopotentiating gene, in particular as defined in claim 4, encoding a selected polypeptide into a tumor cell; and
(b) introducing an exogenous lethal gene as defined in any of claims 1, 5 or 6 into the tumor cell; to produce a tumor cell having the exogenous genes stably maintained in the cell.

11. A tumor cell from a vertebrate organism comprising at least one exogenous immunopotentiating gene, in particular said immunopotentiating gene as defined in claim 4, encoding a selected polypeptide, and an exogenous lethal gene as defined in any of claims 1, 5 or 6, having the genes stably maintained in the cell.

## Claims (Claims for the following Contracting State(s): ES, GR)

1. A composition comprising a tumor cell having been genetically altered by introducing into the cell at least one exogenous immunopotentiating gene, and an exogenous lethal gene including a promoter in operative linkage for use in a method of stimulating in a subject a systemic immune response to a tumor.

2. The composition of claim 1, wherein said tumor is selected from melanoma, adenocarcinoma, and sarcoma.

3. The composition of claim 1 or 2, wherein the tumor cell is derived from a mammal, in particular a human.

4. The composition of any of the preceding claims, wherein the at least one immunopotentiating gene codes for a cytokine selected from interleukin (IL), in particular IL-4; and interferon, in particular gamma interferon; or codes for a foreign antigen selected from a viral protein, in particular the influenza hemagglutinin (HA); a bacterial cell antigen, particularly from *Mycobacterium tuberculosis* and more particularly the 65kD antigen of *M*. *tuberculosis*; and an allogeneic histocompatibility antigen.

5. The composition of any of the preceding claims, wherein said exogenous lethal gene is the gene for Diphtheria toxin A chain.

6. The composition of any of the preceding claims, wherein said promoter is an inducible promoter, particularly the 6-16 interferon promoter.

7. The composition of any of the preceding claims, wherein the exogenous immunopotentiating gene and/or the exogenous lethal gene has been introduced into the cell by transfection with a vector.

8. The composition of claim 7, wherein the transfection of the cell with the vector is mediated by calcium phosphate coprecipitation, liposomes or electroporation.

9. A tumor cell obtained by
(a) introducing at least one exogenous immunopotentiating gene, in particular as defined in claim 4, encoding a selected polypeptide into a tumor cell; and
(b) introducing an exogenous lethal gene as defined in any of claims 1, 5 or 6 into the tumor cell; to produce a tumor cell having the exogenous genes stably maintained in the cell;
for use in a method for gene therapy of cancer of a mammalian subject.

10. A method for producing a tumor cell for gene therapy of cancer comprising:
(a) introducing at least one exogenous immunopotentiating gene, in particular as defined in claim 4, encoding a selected polypeptide into a tumor cell; and
(b) introducing an exogenous lethal gene as defined in any of claims 1, 5 or 6 into the tumor cell; to produce a tumor cell having the exogenous genes stably maintained in the cell.

11. A tumor cell from a vertebrate organism comprising at least one exogenous immunopotentiating gene, in particular said immunopotentiating gene as defined in claim 4, encoding a selected polypeptide, and an exogenous lethal gene as defined in any of claims 1, 5 or 6, having the genes stably maintained in the cell.

12. A method for producing a tumor cell as defined in any of claims 1 to 8, said method comprising the steps of:
(a) introducing into a tumor cell at least one exogenous immunopotentiating gene; and
(b) introducing an exogenous lethal gene including a promoter in operative linkage;
to produce a tumor cell having the exogenous genes stably maintained in the cell.

13. A method for producing a tumor cell from a vertebrate organism as defined in claim 11, said method comprising the steps of:
(a) introducing into a tumor cell at least one exogenous immunopotentiating gene; and
(b) introducing an exogenous lethal gene including a promoter in operative linkage;
to produce a tumor cell having the exogenous genes stably maintained in the cell.

## Patentansprüche (Patentansprüche für folgende(n) Vertragsstaat(en): AT, BE, CH, DE, DK, FR, GB, IT, LI, LU, NL, SE)

1. Zusammensetzung umfassend eine Tumorzelle, die genetisch verändert wurde indem man in die Zelle zumindest ein exogenes immunpotentiierendes Gen, und ein exogenes letales Gen einschließlich eines Promotors in operativer Verknüpfung, eingebracht hat, zur Verwendung in einem Verfahren zum Stimulieren einer systemischen Immunantwort gegen einen Tumor in einem Subjekt.

2. Zusammensetzung nach Anspruch 1, wobei der Tumor ausgewählt ist aus Melanom, Adenokarzinom und Sarkom.

3. Zusammensetzung nach Anspruch 1 oder 2, wobei der Tumor von einem Säugetier, insbesondere einem Menschen, stammt.

4. Zusammensetzung nach irgendeinem der vorangehenden Ansprüche, wobei das zumindest eine immunpotentiierende Gen für ein Zytokin ausgewählt aus Interleukin (IL), insbesondere IL-4; und Interferon, insbesondere γ-Interferon, kodiert; oder für ein fremdes Antigen ausgewählt aus einem viralen Protein, insbesondere dem Influenzahämaglutinin (HA); einem bakteriellen Zellantigen, insbesondere von *Mycobacterium tuberculosis* und ganz besonders dem 65 kD Antigen von *M. tuberculosis,* und einem allogenen Histokompatibilitätsantigen kodiert.

5. Zusammensetzung nach irgendeinem der vorangehenden Ansprüche, wobei das exogene letale Gen das Gen für die A-Kette des Diphterietoxins ist.

6. Zusammensetzung nach irgendeinem der vorangehenden Ansprüche, wobei der Promotor ein induzierbarer Promotor ist, insbesondere der 6-16 Interferon Promotor.

7. Zusammensetzung nach irgendeinem der vorangehenden Ansprüche, wobei das exogene immunpotentiierende Gen und/oder das exogene letale Gen in die Zelle durch Transfektion mit einem Vektor eingebracht wurde.

8. Zusammensetzung nach Anspruch 7, wobei die Transfektion der Zelle mit dem Vektor durch Kalziumphosphat-Kopräzipitation, Liposomen oder Elektroporation vermittelt ist.

9. Eine Tumorzelle, erhalten durch:
a) Einbringen von zumindest einem exogenen immunpotentiierenden Gen, insbesondere wie in Anspruch 4 definiert, welches ein ausgewähltes Polypeptid kodiert, in eine Tumorzelle; und
b) Einbringen eines exogenen letalen Gens wie in einem der Ansprüche 1, 5 oder 6 definiert, in die Tumorzelle; um eine Tumorzelle herzustellen, die die exogenen Gene in der Zelle stabil beibehalten hat;
zur Verwendung in einem Verfahren zur Gentherapie von Krebs eines Säugetiersubj ektes.

10. Verfahren zur Herstellung einer Tumorzelle zur Gentherapie von Krebs umfassend:
a) Einbringen von zumindest einem exogenen immunpotentiierenden Gen, insbesondere wie in Anspruch 4 definiert, welches ein ausgewähltes Polypeptid kodiert, in eine Tumorzelle; und
b) Einbringen eines exogenen letalen Gens wie in einem der Ansprüche 1, 5 oder 6 definiert, in die Tumorzelle; um eine Tumorzelle herzustellen, die die exogenen Gene in der Zelle stabil beibehalten hat.

11. Eine Tumorzelle von einem Wirbeltierorganismus umfassend zumindest ein exogenes immunpotentiierendes Gen, insbesondere das immunpotentiierende Gen wie in Anspruch 4 definiert, welches für ein ausgewähltes Polypeptid kodiert, und ein exogenes letales Gen wie in einem der Ansprüche 1, 5 oder 6 definiert, welche die Gene stabil in der Zelle beibehalten hat.

## Patentansprüche (Patentansprüche für folgende(n) Vertragsstaat(en): ES, GR)

1. Zusammensetzung umfassend eine Tumorzelle, die genetisch verändert wurde indem man in die Zelle zumindest ein exogenes immunpotentiierendes Gen, und ein exogenes letales Gen einschließlich eines Promotors in operativer Verknüpfung, eingebracht hat, zur Verwendung in einem Verfahren zum Stimulieren einer systemischen Immunantwort gegen einen Tumor in einem Subjekt.

2. Zusammensetzung nach Anspruch 1, wobei der Tumor ausgewählt ist aus Melanom, Adenokarzinom und Sarkom.

3. Zusammensetzung nach Anspruch 1 oder 2, wobei der Tumor von einem Säugetier, insbesondere einem Menschen, stammt.

4. Zusammensetzung nach irgendeinem der vorangehenden Ansprüche, wobei das zumindest eine immunpotentiierende Gen für ein Zytokin ausgewählt aus Interleukin (IL), insbesondere IL-4; und Interferon, insbesondere γ-Interferon, kodiert; oder für ein fremdes Antigen ausgewählt aus einem viralen Protein, insbesondere dem Influenzahämaglutinin (HA); einem bakteriellen Zellantigen, insbesondere von *Mycobacterium tuberculosis* und ganz besonders dem 65 kD Antigen von *M. tuberculosis,* und einem allogenen Histokompatibilitätsantigen kodiert.

5. Zusammensetzung nach irgendeinem der vorangehenden Ansprüche, wobei das exogene letale Gen das Gen für die A-Kette des Diphterietoxins ist.

6. Zusammensetzung nach irgendeinem der vorangehenden Ansprüche, wobei der Promotor ein induzierbarer Promotor ist, insbesondere der 6-16 Interferon Promotor.

7. Zusammensetzung nach irgendeinem der vorangehenden Ansprüche, wobei das exogene immunpotentiierende Gen und/oder das exogene letale Gen in die Zelle durch Transfektion mit einem Vektor eingebracht wurde.

8. Zusammensetzung nach Anspruch 7, wobei die Transfektion der Zelle mit dem Vektor durch Kalziumphosphat-Kopräzipitation, Liposomen oder Elektroporation vermittelt ist.

9. Eine Tumorzelle, erhalten durch:
a) Einbringen von zumindest einem exogenen immunpotentiierenden Gen, insbesondere wie in Anspruch 4 definiert, welches ein ausgewähltes Polypeptid kodiert, in eine Tumorzelle; und
b) Einbringen eines exogenen letalen Gens wie in einem der Ansprüche 1, 5 oder 6 definiert, in die Tumorzelle; um eine Tumorzelle herzustellen, die die exogenen Gene in der Zelle stabil beibehalten hat;
zur Verwendung in einem Verfahren zur Gentherapie von Krebs eines Säugetiersubjektes.

10. Verfahren zur Herstellung einer Tumorzelle zur Gentherapie von Krebs umfassend:
a) Einbringen von zumindest einem exogenen immunpotentiierenden Gen, insbesondere wie in Anspruch 4 definiert, welches ein ausgewähltes Polypeptid kodiert, in eine Tumorzelle; und
b) Einbringen eines exogenen letalen Gens wie in einem der Ansprüche 1, 5 oder 6 definiert, in die Tumorzelle; um eine Tumorzelle herzustellen, die die exogenen Gene in der Zelle stabil beibehalten hat.

11. Eine Tumorzelle von einem Wirbeltierorganismus umfassend zumindest ein exogenes immunpotentiierendes Gen, insbesondere das immunpotentiierende Gen wie in Anspruch 4 definiert, welches für ein ausgewähltes Polypeptid kodiert, und ein exogenes letales Gen wie in einem der Ansprüche 1, 5 oder 6 definiert, welche die Gene stabil in der Zelle beibehalten hat.

12. Verfahren zur Herstellung einer wie in irgendeinem der Ansprüche 1 bis 8 definierten Tumorzelle, wobei das Verfahren die Schritte umfasst:
a) Einbringen von zumindest einem exogenen immunpotentiierenden Gen in eine Tumorzelle; und
b) Einbringen eines exogenen letalen Gens einschließlich eines Promotors in operativer Verknüpfung;
um eine Tumorzelle herzustellen, die die exogenen Gene in der Zelle stabil beibehalten hat.

13. Verfahren zur Herstellung einer Tumorzelle aus einem Wirbeltierorganismus wie in Anspruch 11 definiert, wobei das Verfahren die Schritte umfasst:
a) Einbringen von zumindest einem exogenen immunpotentiierenden Gen in eine Tumorzelle; und
b) Einbringen eines exogenen letalen Gens einschließlich eines Promotors in operativer Verknüpfung;
um eine Tumorzelle herzustellen, die die exogenen Gene in der Zelle stabil beibehalten hat.

## Revendications (Revendications pour l'(les) Etat(s) contractant(s) suivant(s): AT, BE, CH, LI, DE, DK, FR, GB, IT, LU, NL, SE)

1. Composition comprenant une cellule tumorale ayant été génétiquement modifiée en introduisant dans la cellule au moins un gène immunopotentiateur exogène, et un gène létal exogène comportant un promoteur dans une liaison fonctionnelle, à utiliser dans une méthode de stimulation chez un sujet d'une réponse immunitaire systémique à une tumeur.

2. Composition selon la revendication 1, dans laquelle ladite tumeur est choisie parmi le mélanome, l'adénocarcinome et le sarcome.

3. Composition selon la revendication 1 ou 2, dans laquelle la cellule tumorale est issue d'un mammifère, en particulier d'un homme.

4. Composition selon l'une quelconque des revendications précédentes, dans laquelle au moins un gène immunopotentiateur code pour une cytokine choisie parmi une interleukine (IL), en particulier IL 4 ; et un interféron, en particulier l'interféron gamma ; ou code pour un antigène étranger choisi parmi une protéine virale, en particulier l'hémagglutinine de la grippe (HA) ; un antigène de cellule bactérienne, en particulier de *Mycobacterium tuberculosis* et plus particulièrement l'antigène de 65 kDa de *M. tuberculosis* ; et un antigène d'histocompatibilité allogénique.

5. Composition selon l'une quelconque des revendications précédentes, dans laquelle ledit gène létal exogène est le gène pour la chaîne A de la toxine diphtérique.

6. Composition selon l'une quelconque des revendications précédentes, dans laquelle ledit promoteur est un promoteur inductible, en particulier le promoteur de l'interféron 6-16.

7. Composition selon l'une quelconque des revendications précédentes, dans laquelle le gène immunopotentiateur exogène et/ou le gène létal exogène a été introduit dans la cellule par transfection à l'aide d'un vecteur.

8. Composition selon la revendication 7, dans laquelle la transfection de la cellule par le vecteur est induite par coprécipitation de phosphate de calcium, des liposomes ou électroporation.

9. Cellule tumorale obtenue
(a) en introduisant au moins un gène immunopotentiateur exogène, en particulier tel que défini dans la revendication 4, codant pour un polypeptide sélectionné dans une cellule tumorale ; et
(b) en introduisant un gène létal exogène tel que défini dans l'une quelconque des revendications 1, 5 ou 6 dans la cellule tumorale : pour produire une cellule tumorale, les gènes exogènes étant maintenus de manière stable dans la cellule ;
à utiliser dans une méthode de thérapie génique du cancer chez un sujet mammifère.

10. Procédé de production d'une cellule tumorale pour la thérapie génique d'un cancer comprenant :
(a) l'introduction d'au moins un gène immunopotentiateur exogène, en particulier tel que défini dans la revendication 4, codant pour un polypeptide sélectionné dans une cellule tumorale ; et
(b) l'introduction d'un gène létal exogène tel que défini dans l'une quelconque des revendications 1, 5 ou 6 dans la cellule tumorale ; pour produire une cellule tumorale, les gènes exogènes étant maintenus de manière stable dans la cellule.

11. Cellule tumorale issue d'un organisme vertébré comprenant au moins un gène immunopotentiateur exogène, en particulier ledit gène immunopotentiateur tel que défini dans la revendication 4, codant pour un polypeptide sélectionné, et un gène létal exogène tel que défini dans l'une quelconque des revendications 1, 5 ou 6, les gènes étant maintenus de manière stable dans la cellule.

## Revendications (Revendications pour l'(les) Etat(s) contractant(s) suivant(s): ES, GR)

1. Composition comprenant une cellule tumorale ayant été génétiquement modifiée en introduisant dans la cellule au moins un gène immunopotentiateur exogène, et un gène létal exogène comportant un promoteur dans une liaison fonctionnelle, à utiliser dans une méthode de stimulation chez un sujet d'une réponse immunitaire systémique à une tumeur.

2. Composition selon la revendication 1, dans laquelle ladite tumeur est choisie parmi le mélanome, l'adénocarcinome et le sarcome.

3. Composition selon la revendication 1 ou 2, dans laquelle la cellule tumorale est issue d'un mammifère, en particulier d'un homme.

4. Composition selon l'une quelconque des revendications précédentes, dans laquelle au moins un gène immunopotentiateur code pour une cytokine choisie parmi une interleukine (IL), en particulier IL 4 ; et un interféron, en particulier l'interféron gamma ; ou code pour un antigène étranger choisi parmi une protéine virale, en particulier l'hémagglutinine de la grippe (HA) ; un antigène de cellule bactérienne, en particulier de *Mycobacterium tuberculosis* et plus particulièrement l'antigène de 65 kDa de *M. tuberculoses* ; et un antigène d'histocompatibilité allogénique.

5. Composition selon l'une quelconque des revendications précédentes, dans laquelle ledit gène létal exogène est le gène pour la chaîne A de la toxine diphtérique.

6. Composition selon l'une quelconque des revendications précédentes, dans laquelle ledit promoteur est un promoteur inductible, en particulier le promoteur de l'interféron 6-16.

7. Composition selon l'une quelconque des revendications précédentes, dans laquelle le gène immunopotentiateur exogène et/ou le gène létal exogène a été introduit dans la cellule par transfection à l'aide d'un vecteur.

8. Composition selon la revendication 7, dans laquelle la transfection de la cellule par le vecteur est induite par coprécipitation de phosphate de calcium, des liposomes ou électroporation.

9. Cellule tumorale obtenue
(a) en introduisant au moins un gène immunopotentiateur exogène, en particulier tel que défini dans la revendication 4, codant pour un polypeptide sélectionné dans une cellule tumorale ; et
(b) en introduisant un gène létal exogène tel que défini dans l'une quelconque des revendications 1, 5 ou 6 dans la cellule tumorale ; pour produire une cellule tumorale, les gènes exogènes étant maintenus de manière stable dans la cellule ;
à utiliser dans une méthode de thérapie génique du cancer chez un sujet mammifère.

10. Procédé de production d'une cellule tumorale pour la thérapie génique d'un cancer comprenant :
(a) l'introduction d'au moins un gène immunopotentiateur exogène, en particulier tel que défini dans la revendication 4, codant pour un polypeptide sélectionné dans une cellule tumorale ; et
(b) l'introduction d'un gène létal exogène tel que défini dans l'une quelconque des revendications 1, 5 ou 6 dans la cellule tumorale ; pour produire une cellule tumorale, les gènes exogènes étant maintenus de manière stable dans la cellule.

11. Cellule tumorale issue d'un organisme vertébré comprenant au moins un gène immunopotentiateur exogène, en particulier ledit gène immunopotentiateur tel que défini dans la revendication 4, codant pour un polypeptide sélectionné, et un gène létal exogène tel que défini dans l'une quelconque des revendications 1, 5 ou 6, les gènes étant maintenus de manière stable dans la cellule.

12. Procédé de production d'une cellule tumorale selon l'une quelconque des revendications 1 à 8, ledit procédé comportant les étapes consistant à :
(a) introduire dans une cellule tumorale au moins un gène immunopotentiateur exogène ; et
(b) introduire un gène létal exogène comportant un promoteur dans une liaison fonctionnelle ;
afin de produire une cellule tumorale, les gènes exogènes étant maintenus de manière stable dans la cellule.

13. Procédé de production d'une cellule tumorale à partir d'un organisme vertébré selon la revendication 11, ledit procédé comportant les étapes consistant à :
(a) introduire dans une cellule tumorale au moins un gène immunopotentiateur exogène ; et
(b) introduire dans un gène létal exogène comportant un promoteur dans une liaison fonctionnelle ;
afin de produire une cellule tumorale, les gènes exogènes étant maintenus de manière stable dans la cellule.
